# EUROPÄISCHE PATENTSCHRIFT

(11) **EP 2 398 764 B1**
(45) Veröffentlichungstag und Bekanntmachung des Hinweises auf die Patenterteilung: **26.11.2014**
(21) Anmeldenummer: 10711611.3
(22) Anmeldetag: 18.02.2010
(51) Int. Cl.: C07C 209/68, C07C 211/52, C07C 251/24, C07C 257/12, C07B 37/04

(54) **VERFAHREN ZUR HERSTELLUNG VON 2-AMINOBIPHENYLEN**
METHOD FOR MANUFACTURING 2-AMINOBIPHENYLS
PROCÉDÉ DE FABRICATION DE 2-AMINOBIPHENYLÈNES

(30) Priorität: 19.02.2009 EP 09153239
(43) Veröffentlichungstag der Anmeldung: 28.12.2011
(73) Patentinhaber: BASF SE, 67056 Ludwigshafen (DE)
(72) Erfinder: ZIERKE, Thomas, 67459 Böhl-Iggelheim (DE); MAYWALD, Volker, 67071 Ludwigshafen (DE); SMIDT, Sebastian, Peer, 68723 Oftersheim (DE)
(86) Internationale Anmeldenummer: PCT/EP2010/052033
(87) Internationale Veröffentlichungsnummer: WO 2010/094736

(56) Entgegenhaltungen:
- EP-A1- 0 595 150
- WO-A1-2005/123690
- GB-A- 796 951
- XU, H. ET AL.: "Palladium-phosphinous acid-catalyzed cross-coupling of aryl and acyl halides with aryl-, alkyl-, and vinylzinc reagents" J. ORG. CHEM., Bd. 73, 9. April 2008 (2008-04-09), Seiten 7638-7650, XP002558978
- DAI, C. ET AL.: "The first general method for Palladium-catalyzed Negishi cross-coupling of aryl and vinyl chlorides: Use of commercially available Pd(P(t-Bu)3)2 as a catalyst" J. AM. CHEM. SOC., Bd. 123, 2001, Seiten 2719-2724, XP002558979 in der Anmeldung erwähnt
- ORGAN, M.G. ET AL.: "A user-friendly, all-purpose Pd-NHC (NHC = N-Heterocyclic Carbene) precatalyst for the Negishi reaction: A step towards a universal cross-coupling catalyst" CHEM. EUR. J., Bd. 12, 2006, Seiten 4749-4755, XP002558980 in der Anmeldung erwähnt
- LI, G.Y.: "Highly active, air-stable Palladium catalysts for the C-C and C-S bond-forming reactions of vinyl and aryl chlorides: Use of commercially available [(t-Bu)2P(OH)]2PdCl2, [(t-Bu)2P(OH)PdCl2]2, and [[(t-Bu)2PO...H...OP(t-Bu)2]PdCl]2 as catalysts" J. ORG. CHEM., Bd. 67, 2002, Seiten 3643-3650, XP002558981
- JENSEN, A.E. ET AL.: "Preparation of 2-arylated-1,4-phenylenediamines by palladium-catalyzed cross-coupling reactions" JOURNAL OF ORGANOMETALLIC CHEMISTRY, Bd. 653, Nr. 1-2, 2002, Seiten 122-128, XP004361513 ISSN: 0022-328X
- MANOLIKAKES, G. ET AL.: "Palladium- and Nickel-catalyzed cross-couplings of unsaturated halides bearing relatively acidic protons with organozinc reagents" J. ORG. CHEM., Bd. 73, 10. April 2008 (2008-04-10), Seiten 8422-8436, XP002558982
- ALBERT, J. ET AL.: "Steric and electronic effects on E/Z composition of exocyclic cyclopalladated N-benzylideneamines" JOURNAL OF ORGANOMETALLIC CHEMISTRY, Bd. 545-546, 1997, Seiten 131-137, XP004103327 ISSN: 0022-328X
- LEARDINI, R. ET AL.: "A new and convenient synthesis of phenanthridines" SYNTHESIS, Bd. 1985, Nr. 1, 1985, Seiten 107-110, XP002558983
- NIWA, H.: "Studies on the syntheses of diphenyl derivatives. (VI) On the amidines" TOHOKU YAKKA DAIGAKU KENKYU NEMPO, TOHOKU YAKKA DAIGAKU, SENDAI, JP, Bd. 4, 1957, Seiten 79-89, XP009126695 ISSN: 0495-7342

## Beschreibung

Die vorliegende Erfindung betrifft ein Verfahren zur Herstellung von 2-Aminobiphenylen der Formel I, 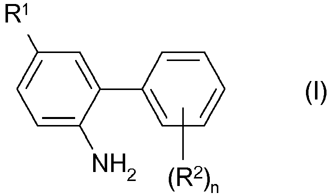 worin
- n: für 0, 1, 2 oder 3 steht,
- R¹: für Wasserstoff, Cyano oder Fluor steht, und
jedes R² jeweils unabhängig voneinander ausgewählt ist unter Cyano, Fluor, C₁-C₄-Alkyl, C₁-C₄-Fluoralkyl, C₁-C₄-Alkoxy, C₁-C₄-Fluoralkoxy, C₁-C₄-Alkylthio und C₁-C₄-Fluoralkylthio.

Die Erfindung betrifft auch ein Verfahren zur Herstellung von Pyrrazolcarboxamiden solcher 2-Aminobiphenyle.

2-Aminobiphenyle der Formel I sind wichtige Vorstufen für Aryl- und Hetarylcarboxamide, welche insbesondere als Fungizide Verwendung finden. Solche Fungizide sind beispielsweise in WO 2006/087343, WO 2005/123690, EP 0589301 und EP 0545099 offenbart. Zu ihren prominenten Vertretern gehören Boscalid (2-Chlor-N-(4'-chlorbiphenyl-2-yl)-pyridin-3-carboxamid) und Bixafen (N-(3',4'-Dichlor-5-fluorbiphenyl-2-yl)-3-(difluormethyl)-1-methylpyrazol-4-carboxamid)

Zur Herstellung von substituierten 2-Aminobiphenylen der Formel I hat sich ein in WO 97/33846 beschriebenes Verfahren bewährt, das auf der Kupplung von 2-Nitrochlorbenzol mit halogensubstituierten aromatischen Boronsäuren zu den entsprechend substituierten 2-Nitrobiphenylen beruht, welche anschließend zu 2-Aminobiphenylen reduziert werden. Hierbei handelt es sich allerdings um einen vielstufigen Syntheseweg, der folglich zeit- und kostenaufwendig ist. So erfordert er sowohl die Bereitstellung der Boronsäuren, die üblicherweise in drei Stufen, ausgehend von Halogenaromaten über Grignardverbindungen, Boronsäureestern und nachfolgender Hydrolyse, hergestellt werden als auch die abschließende Hydrierung der Nitrofunktion.

Palladium-katalysierte Kreuzkupplungen von Halogenaromaten mit Zinkorganylen, sogenannte Negishi-Kupplungen, sind seit längerem bekannt (E. Negishi et al., J. Org. Chem. 1977, 42; E. Negishi in "Metal-catalyzed Cross-Coupling Reactions; F. Diederich, P. J. Stang (Hsg.) Wiley-VCH, Weinheim, 1998, S. 1821). Über die Umsetzung der reaktionsträgeren Chloraromaten im Sinne einer Negishi-Kupplung wurde in Einzelfällen berichtet (C. Dai, G. C. Fu, J. Am. Chem. Soc. 2001, 123, 2719).

Für die Herstellung von 2-Aminobiphenylen wurde die Negishi-Kupplung bislang nur ausgehend von den besonders reaktiven Iodanilinderivaten verwendet. So sind Umsetzungen von 2- bzw. 3-Jodanilin sowie von 3-lodoanthranilonitril mit Arylzinkjodiden bekannt (N. Jeong et al., Bull. Korean Chem. Soc. 2000, 21, 165; J. B. Campbell et al., Synth. Comm. 1989, 19, 2265). Um gute Kupplungsausbeuten zu erzielen, mussten dabei allerdings große Überschüsse an Zinkorganyl und 5 bis 6 mol-% Palladiumkatalysator eingesetzt werden, was die wirtschaftliche Bedeutung dieser Verfahren für viele Anwendungsfälle stark einschränkt.

Jensen et al., J. Organometallic Chem. 2002, 653, 122, beschreiben die Herstellung von 2-Arylsubstituierten 1,4-Phenylendiaminen ausgehend von zweifach geschütztem 2-Iodo-1,4-phenylendiamin über eine Palladium-katalysierte Kreuzkupplungsreaktion.

Li et al., J. Org. Chem. 2002, 67, 3643, beschreiben hochreaktive luftunempfindliche Palladium-Katalysatoren zur Knüpfung von C-C und C-S Bindungen von Vinyl- und Arylchloriden.

Xu et al., J. Org. Chem. 2008, 73, 7638, beschreiben verschiedene Palladium-Hydroxyphosphin Verbindungen, die als Katalysatoren für Kreuzkupplungsreaktionen von Aryl- oder Acylhaliden mit aliphatischen und Arylzinkchloriden verwendet werden können.

Manolikakes et al., J. Org. Chem. 2008, 73, 8422, beschreiben ebenfalls die Verwendung von Arylzinkjodiden für Synthesen von Aminobiphenylen mittels Palladium-katalysierter Kreuzkupplungen. Als Arylhalogenid-Komponente setzten sie hierfür Arylbromide ein, da sich Arylchloride in ihren Händen als ungeeignet herausgestellt hatten. Außerdem wurden für die beschriebenen Synthesen wiederum große Überschüsse an Zinkorganyl benötigt.

Der vorliegenden Erfindung lag die Aufgabe zu Grunde, einfach und im technischen Maßstab durchzuführende Verfahren zur Herstellung von substituierten 2-Aminobiphenylen sowie zur Herstellung von davon abgeleiteten Pyrrazolcarboxamide bereitzustellen. Diese Verfahren sollten zudem kostengünstig sein und auf selektiven Umsetzungen beruhen. So soll auch ein Zugang zu substituierten 2-Aminobiphenylen ausgehend von weniger reaktiven 2-Chloranilinen, die jedoch gegenüber den entsprechenden 2-Bromanilinen in der Regel leichter erhältlich und/oder preiswerter sind, gefunden werden.

Die Aufgabe wird durch die im Folgenden näher beschriebenen Verfahren gelöst.

Gegenstand der vorliegenden Erfindung ist ein Verfahren zur Herstellung der eingangs definierten substituierten 2-Aminobiphenyle der Formel I, umfassend folgende Schritte:
(i) Umsetzung einer Anilinverbindung der Formel II mit einer Zinkorganyl-Verbindung der Formel III, 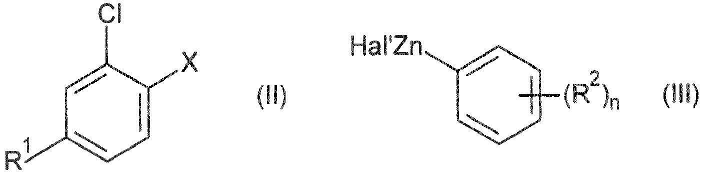 worin
   Hal' für Brom oder Chlor steht,
   n, R¹ und R² die oben angegebenen Bedeutungen haben, und
   X für NH₂ oder einen Rest X¹ oder X² steht, 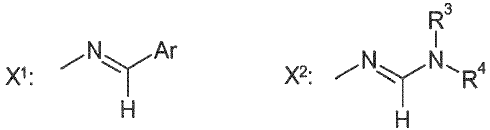 worin
   - Ar: für Phenyl steht, das gegebenenfalls 1, 2, oder 3 Substituenten trägt, die ausgewählt sind unter C₁-C₄-Alkyl und C₁-C₄-Alkoxy, und
   - R³ und R⁴: unabhängig voneinander für C₁-C₆-Alkyl stehen,
   in Gegenwart eines Palladium-Katalysators umfassend Palladium und einen oder mehrere Komplexliganden,
   und, wenn X in Formel II für einen Rest X¹ oder X² steht,
(ii) Umsetzung des in Schritt (i) erhaltenen Produkts zu einem 2-Aminobiphenyl der Formel I.

Das erfindungsgemäße Verfahren ist mit einer Reihe von Vorteilen verbunden. So ermöglicht das erfindungsgemäße Verfahren, 2-Aminobiphenyle der Formel I kostengünstig, in guten bis sehr guten Ausbeuten und mit hohen Selektivitäten herzustellen. Insbesondere erlaubt es die Herstellung von 2-Aminobiphenylen aus den weniger reaktiven Chloranilinverbindungen II.

Im Rahmen der vorliegenden Erfindung haben die generisch verwendeten Begriffe die folgenden Bedeutungen:

Das Präfix Cₓ-C_{y} bezeichnet im jeweiligen Fall die Anzahl möglicher Kohlenstoffatome.

Der Begriff "Halogen" bezeichnet jeweils Fluor, Brom, Chlor oder Iod, speziell Fluor, Chlor oder Brom.

Der Begriff "C₁-C₄-Alkyl" bezeichnet einen linearen oder verzweigten Alkylrest, umfassend 1 bis 4 Kohlenstoffatome, wie Methyl, Ethyl, Propyl, 1-Methylethyl (Isopropyl), Butyl, 1-Methylpropyl (sec-Butyl), 2-Methylpropyl (Isobutyl) oder 1,1-Dimethylethyl (tert-Butyl).

Der Begriff "C₁-C₆-Alkyl" bezeichnet einen linearen oder verzweigten Alkylrest umfassend 1 bis 6 Kohlenstoffatome. Beispiele sind neben den für C₁-C₄-Alkyl genannten Resten Pentyl, Hexyl, 2,2-Dimethylpropyl, 2-Methylbutyl, 3-Ethylbutyl, 4-Methylpentyl und Stellungsisomere davon.

Der Begriff "C₁-C₄-Fluoralkyl", wie hierin und in den Fluoralkyleinheiten von C₁-C₄-Fluoralkoxy und C₁-C₄-Fluoralkylthio verwendet, beschreibt geradkettige oder verzweigte Alkylgruppen mit 1 bis 4 Kohlenstoffatomen, wobei die Wasserstoffatome dieser Gruppen teilweise oder vollständig durch Fluoratome ersetzt sind. Beispiele hierfür sind Fluormethyl, Difluormethyl, Trifluormethyl, 1-Fluorethyl, 2-Fluorethyl, 2,2-Difluorethyl, 2,2,2-Trifluorethyl, Pentafluorethyl, 3,3,3-Trifluorprop-1-yl, 1,1,1-Trifluorprop-2-yl, Heptafluorisopropyl, 1-Fluorbutyl, 2-Fluorbutyl, 3-Fluorbutyl, 4-Fluorbutyl, 4,4,4-Trifluorbutyl, Fluor-tert.-butyl und dergleichen.

Der Begriff "Halomethyl", beschreibt Methylgruppen deren Wasserstoffatome teilweise oder vollständig durch Halogenatome ersetzt sind. Beispiele hierfür sind Chlormethyl, Brommethyl, Dichlormethyl, Trichlormethyl, Fluormethyl, Difluormethyl, Trifluormethyl, Chlorofluormethyl, Dichlorfluormethyl, Chlordifluormethyl, Dibrommethyl, Tribrommethyl, Chlorobrommethyl, Dichlorbrommethyl, Chlordibrommethyl und dergleichen.

Der Begriff "C₁-C₄-Alkoxy" bezeichnet geradkettige oder verzweigte gesättigte Alkylgruppen, umfassend 1 bis 4 Kohlenstoffatome, die über ein Sauerstoffatom gebunden sind. Beispiele für C₁-C₄-Alkoxy sind Methoxy, Ethoxy, n-Propoxy, 1-Methylethoxy (Isopropoxy), n-Butoxy, 1-Methylpropoxy (sec-Butoxy), 2-Methylpropoxy (Isobutoxy) und 1,1-Dimethylethoxy (tert-Butoxy).

Der Begriff "C₁-C₄-Fluoralkoxy" beschreibt geradkettige oder verzweigte gesättigte Fluoralkylgruppen, umfassend 1 bis 4 Kohlenstoffatome, die über ein Sauerstoffatom gebunden sind. Beispiele hierfür sind Fluormethoxy, Difluormethoxy, Trifluormethoxy, 1-Fluorethoxy, 2-Fluorethoxy, 2,2-Difluorethoxy, 2,2,2-Trifluorethoxy, 1,1,2,2-Tetrafluorethoxy, Pentafluorethoxy, 3,3,3-Trifluorprop-1-oxy, 1,1,1-Trifluorprop-2-oxy, 1-Fluorbutoxy, 2-Fluorbutoxy, 3-Fluorbutoxy, 4-Fluorbutoxy und dergleichen.

Der Begriff "C₁-C₄-Alkylthio" bezeichnet geradkettige oder verzweigte gesättigte Alkylgruppen, umfassend 1 bis 4 Kohlenstoffatome, die über ein Schwefelatom gebunden sind. Beispiele für C₁-C₄-Alkylthio sind Methylthio, Ethylthio, n-Propylthio, 1-Methylethylthio (Isopropylthio), n-Butylthio, 1-Methylpropylthio (sec-Butylthio), 2-Methylpropylthio (Isobutylthio) und 1,1-Dimethylethylthio (tert-Butylthio).

Der Begriff "C₁-C₄-Fluoralkylthio" beschreibt geradkettige oder verzweigte gesättigte Fluoralkylgruppen, umfassend 1 bis 4 Kohlenstoffatome, die über ein Schwefelatom gebunden sind. Beispiele hierfür sind Fluormethylthio, Difluormethylthio, Trifluormethylthio, 1-Fluorethylthio, 2-Fluorethylthio, 2,2-Difluorethylthio, 2,2,2-Trifluorethylthio, 1,1,2,2-Tetrafluorethylthio, Pentafluorethylthio, 3,3,3-Trifluorprop-1-ylthio, 1,1,1-Trifluorprop-2-ylthio, 1-Fluorbutylthio, 2-Fluorbutylthio, 3-Fluorbutylthio, 4-Fluorbutylthio und dergleichen.

Der Begriff "Aryl" bezeichnet carbocyclische aromatische Reste mit 6 bis 14 Kohlenstoffatomen. Beispiele hierfür umfassen Phenyl, Naphthyl, Fluorenyl, Azulenyl, Anthracenyl und Phenanthrenyl. Bevorzugt steht Aryl für Phenyl oder Naphthyl und insbesondere Phenyl.

Der Begriff "Hetaryl" bezeichnet aromatische Reste mit 1 bis 4 Heteroatomen, die ausgewählt sind unter O, N und S. Beispiele hierfür sind 5- und 6-gliedrige Hetarylreste mit 1,2,3 oder 4 Heteroatomen ausgewählt unter O, S und N, wie Pyrrolyl, Furanyl, Thienyl, Pyrazolyl, Imidazolyl, Oxazolyl, Isoxazolyl, Thiazolyl, Isothiazolyl, Triazolyl, Tetrazolyl, Pyridyl, Pyrazinyl, Pyridazinyl, Pyrimidyl und Triazinyl.

In den Verbindungen der Formeln I, Ia, Ib, III und IV steht n bevorzugt für 1, 2 oder 3 und insbesondere bevorzugt für 3. Wenn n für 1 steht, befindet sich R² vorzugsweise in para- oder meta-Position zur Biphenyl- bzw. Zinkbindung und wenn n für 2 oder 3 steht, befindet sich R² vorzugsweise in para- und meta-Position zur Biphenyl- bzw. Zinkbindung.

In den Verbindungen der Formeln I, Ia, Ib, II und IV steht R¹ bevorzugt für Wasserstoff oder Fluor und insbesondere bevorzugt für Wasserstoff.

In den Verbindungen der Formeln I, Ia, Ib, III und IV steht R² bevorzugt für Fluor, C₁-C₄-Alkyl oder C₁-C₄-Fluoralkyl und insbesondere bevorzugt für Fluor.

In den Verbindungen der Formel III steht Hai' bevorzugt für Chlor.

In den Verbindungen der Formel II steht X bevorzugt für X¹ oder X², wobei in den Definitionen von X¹ und X² sowie in den Verbindungen der Formeln (la) und (Ib) der Substituent Ar vorzugsweise für unsubstituiertes Phenyl steht und die Substituenten R³ und R⁴ vorzugsweise für Methyl stehen.

In den Verbindungen der Formeln IV und V steht R⁵ bevorzugt für Methyl oder Fluormethyl und insbesondere bevorzugt für Methyl, Difluormethyl oder Trifluormethyl.

Die im Folgenden beschriebenen erfindungsgemäßen Umsetzungen werden in für derartige Reaktionen üblichen Reaktionsgefäßen durchgeführt, wobei die Reaktionsführung sowohl kontinuierlich, semi-kontinuierlich als auch diskontinuierlich ausgestaltet werden kann. In der Regel wird man die jeweiligen Reaktionen unter Atmosphärendruck durchführen. Die Reaktionen können jedoch auch unter vermindertem oder erhöhtem Druck durchgeführt werden.

Bei der Umsetzung in Schritt (i) des erfindungsgemäßen Verfahrens zur Herstellung substituierter 2-Aminobiphenyle I handelt es sich um eine Negishi-Kreuzkupplung. Die Umsetzung erfolgt, indem man die Ausgangsverbindungen, d.h. eine Anilinverbindung II und eine Zinkorganylverbindung III sowie einen Palladium-Katalysator, vorzugsweise in einem Lösungsmittel, unter geeigneten Reaktionsbedingungen in Kontakt bringt.

In der Regel wird Schritt (i) unter Temperaturkontrolle durchgeführt. Die Negishi-Kupplung erfolgt üblicherweise in einem verschlossenem oder unverschlossenem Reaktionsgefäß mit Rühr- und Heizvorrichtung.

Die Reaktanden können prinzipiell in beliebiger Reihenfolge miteinander in Kontakt gebracht werden. So kann beispielsweise die Anilinverbindung II, gegebenenfalls in einem Lösungsmittel gelöst oder dispergiert, vorgelegt und mit dem Zinkorganyl III versetzt werden oder umgekehrt das Zinkorganyl III, gegebenenfalls in einem Lösungsmittel gelöst oder dispergiert, vorgelegt und mit der Anilinverbindung versetzt werden.

Alternativ können auch die beiden Edukte gleichzeitig in das Reaktionsgefäß gegeben werden. Der Palladium-Katalysator kann vor oder nach der Zugabe eines der Edukte oder aber zusammen mit einem der Edukte, entweder in einem Lösungsmittel oder in Substanz, zugegeben werden.

Es hat sich als zweckmäßig erwiesen, das Zinkorganyl III, vorzugsweise in einem Lösungsmittel, vorzulegen und den Palladium-Katalysator, bzw. eine Palladiumquelle und einen oder mehrere komplexierende Liganden aus denen der aktive Katalysator gebildet wird, sowie die Anilinverbindung II hinzuzugeben.

Geeignete Lösungsmittel hängen im Einzelnen von der Wahl der jeweiligen Reaktionspartner und Reaktionsbedingungen ab. Es hat sich allgemein als vorteilhaft erwiesen, als Lösungsmittel für die Umsetzung der Verbindungen (II) und (III) ein aprotisches organisches Lösungsmittel zu verwenden. Als aprotische organische Lösungsmittel kommen hier beispielsweise aliphatische C₃-C₆-Ether, wie Dimethoxyethan, Diethylenglykoldimethylether, Dipropylether, Methylisobutylether, tert.-Butylmethylether und tert.-Butylethylether, aliphatische Kohlenwasserstoffe, wie Pentan, Hexan, Heptan und Octan sowie Petrolether, cycloaliphatische Kohlenwasserstoffe, wie Cyclopentan und Cyclohexan, alicyclische C₃-C₆-Ether, wie Tetrahydrofuran, Tetrahydropyran, 2-Methyltetrahydrofuran, 3-Methyltetrahydrofuran und Dioxan, aromatische Kohlenwasserstoffe, wie Benzol, Toluol, die Xylole und Mesitylen, kurzkettige Ketone, wie Aceton, Ethylmethylketon und iso-Butylmethylketon, Amide wie Dimethylformamid, Dimethylacetamid und *N*-Methylpyrrolidon, Dimethylsulfoxid, Acetonitril, oder Mischungen dieser Lösungsmittel untereinander in Frage.

Vorzugsweise umfasst das Lösungsmittel für die Umsetzung in Schritt (i) wenigstens einen Ether, insbesondere einen aliphatischen oder alicyclischen C₃-C₆-Ether, oder eine Mischung von wenigstens einem der vorgenannten Ether mit wenigstens einem Amid, insbesondere *N*-Methylpyrrolidon. Bevorzugt machen die vorgenannten Lösungsmittel wenigstens 70 Gew.-%, insbesondere wenigstens 90 Gew.-% der gesamten Lösungsmittelmenge aus. Es ist jedoch möglich, die vorgenannten Lösungsmittel mit Kohlenwasserstoffen, insbesondere aromatischen Kohlenwasserstoffen zu verdünnen, wobei der Anteil an Kohlenwasserstoffen vorzugsweise nicht mehr als 30 Gew.-%, insbesondere nicht mehr als 10 Gew.-% der gesamten Lösungsmittelmenge ausmacht. Von den vorgenannten Mischungen sind Mischungen von Tetrahydrofuran mit N-Methylpyrrolidon besonders bevorzugt, wobei vorzugsweise das Zinkorganyl III in THF vorgelegt wird, dann der Palladiumkatalysator und *N*-Methylpyrrolidon, entweder zusammen oder nacheinander, zugefügt werden und im Anschluss daran die Anilinverbindung II zugegeben wird. Im Falle der Umsetzung einer Anilinverbindung II bei der X für NH₂ steht, liegt das Gewichtsverhältnis von Tetrahydrofuran zu *N*-Methylpyrrolidon vorzugsweise im Bereich von 5:1 bis 1 : 7 und besonders bevorzugt im Bereich von 3 : 1 bis 1 : 4. Im Falle der Umsetzung einer Anilinverbindung II bei der X für X¹ oder X² steht, liegt das Gewichtsverhältnis von Tetrahydrofuran zu *N*-Methylpyrrolidon vorzugsweise im Bereich von 15 : 1 bis 1 : 1 und besonders bevorzugt im Bereich von 10 : 1 bis 3 : 1.

Die Gesamtmenge des in Schritt (i) des erfindungsgemäßen Verfahrens eingesetzten Lösungsmittels liegt typischerweise im Bereich von 100 bis 3000 g und bevorzugt im Bereich von 150 bis 2000 g, bezogen auf 1 Mol des Zinkorganyls III.

Vorzugsweise werden Lösungsmittel verwendet, die im Wesentlichen wasserfrei sind, d.h. einen Wassergehalt von weniger als 1000 ppm und insbesondere nicht mehr als 100 ppm aufweisen.

Gemäß einer bevorzugten Ausführungsform der Erfindung setzt man in Schritt (i) die Anilinverbindung der Formel II, falls X für NH₂ steht, in einer Menge von 0,1 bis 1 Mol, besonders bevorzugt von 0,3 bis 0,9 Mol und insbesondere von 0,5 bis 0,8 Mol, jeweils bezogen auf 1 Mol des Zinkorganyls der Formel III, ein. Falls hingegen X für X¹ oder X² steht, wird die Anilinverbindung II vorzugsweise in einer Menge von 0,5 bis 1,5 Mol, besonders bevorzugt von 0,7 bis 1,3 Mol und insbesondere von 0,9 bis 1,1 Mol, jeweils bezogen auf 1 Mol des Zinkorganyls III, eingesetzt.

Wie bereits gesagt, wird in einer bevorzugten Ausführungsform das Zinkorganyl III für die Umsetzung gemäß Schritt (i) in einem Lösungsmittel gelöst oder dispergiert vorgelegt. Das Zinkorganyl kann dazu sowohl direkt eingesetzt werden oder in situ gebildet werden. Die in situ-Bildung erfolgt üblicherweise analog bekannter Verfahrensweisen, wie beispielsweise von Fu et al., J. Am. Chem. Soc. 2001, 123, 2721 beschrieben, durch Transmetallierung eines metallorganischen Vorläufers des Zinkorganyls III mit einer in etwa äquimolaren Menge eines Zinksalzes, vorzugsweise Zinkchlorid oder Zinkbromid und insbesondere Zinkchlorid. Als metallorganische Vorläufer dienen hier vorzugsweise die entsprechenden Lithium-organischen Verbindungen oder die entsprechenden Magnesium-organischen Grignardverbindungen. Die Struktur dieser bevorzugten Vorläuferverbindungen lässt sich aus der Formel III herleiten, indem man den Substituenten "Hal'Zn" durch "Li" bzw. "Hal"Mg" ersetzt, wobei Hal" vorzugsweise für Chlor oder Brom steht. Die Umsetzung zur in situ-Bildung wird vorzugsweise in einem Lösungmittel vorgenommen, bei dem es sich in der Regel um eines der zuvor genannten aprotischen Lösungsmittel, besonders bevorzugt um Ether und insbesondere um THF handelt. Typischerweise wird eine Vorläuferverbindung mit einem Zinksalz bei einer Temperatur von in der Regel 10 bis 50 °C und bevorzugt von 15 bis 35°C über einen Zeitraum von 1 bis 60 Minuten und bevorzugt von 5 bis 30 Minuten umgesetzt. Das so erhaltene, üblicherweise in einem Lösungsmittel vorliegende Zinkorganyl III, wird gemäß Schritt (i) zur Reaktion gebracht.

Es wird vermutet, dass der Mechanismus der Umsetzung in Schritt (i) dem für die Negishi-Kreuzkupplung vorgeschlagenen Mechanismus entspricht. Demnach wird zunächst im ersten Schritt, der oxidativen Addition, durch die Insertion von Palladium(0) des Katalysators in die R-Hal-Bindung der Anilinverbindung II, ein Organylpalladium(II)-Komplex gebildet. Im zweiten Schritt erfolgt die Übertragung des Organylrestes der zinkorganischen Verbindung III auf das Palladium(II). Im darauffolgenden Schritt, der trans/cis-Isomerisierung, lagern sich die beiden neu in den Palladiumkomplex eingetretenen Organylliganden so um, dass sie sich zueinander in der cis-Position befinden. Im letzten Schritt, der reduktiven Eliminierung, werden die beiden Reste, unter Reduktion von Palladium(II) zu Palladium(0), durch C-C-Verknüpfung miteinander gekuppelt.

Wie zuvor erläutert wird in Schritt (i) eine Anilinverbindung II entweder mit freier Aminofunktion (X steht für NH₂) oder, gemäß einer bevorzugten Ausführungsform der Erfindung, mit geschützter Aminofunktion, wobei X entweder für X¹ oder X² steht, eingesetzt. Bei letzteren handelt es sich um das Iminderivat IIa bzw. um das Amidinderivat IIb der Anilinverbindung II mit freier NH₂-Gruppe: 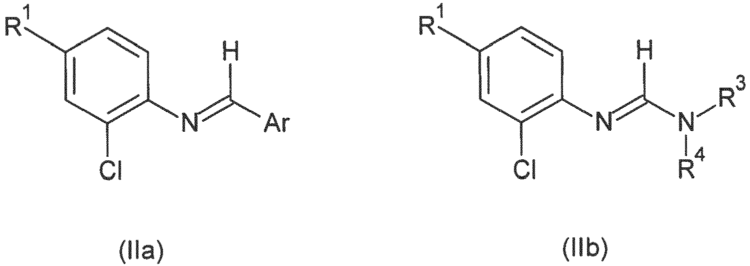 wobei R¹,R³, R⁴ und Ar die oben angegebenen Bedeutungen haben.

Im erfindungsgemäßen Verfahren zur Herstellung von Verbindungen I hat der Einsatz der Anilinverbindungen II mit freier Aminofunktion gegenüber den geschützten Verbindungen IIa oder IIb den Vorteil, dass zwei Syntheseschritte weniger erforderlich sind. Dabei handelt es sich zum einen um die Überführung einer Anilinverbindung II mit freier Aminofunktion in ein Iminderivat der Formel IIa bzw. in ein Amidinderivat der Formel IIb und zum anderen um die Freisetzung der Aminogruppe im Schritt (ii) des erfindungsgemäßen Verfahrens. Andererseits weist der Einsatz der geschützten Verbindungen IIa oder IIb gegenüber dem Einsatz des freien Amins II (X = NH₂) überraschende Vorteile auf. So führen die Umsetzungen des freien Amins in Schritt (i), insbesondere bei höheren Temperaturen, die aufgrund einer geringeren Reaktivität der eingesetzten Edukte oder des eingesetzten Palladiumkatalysators erforderlich sein mögen, unter Umständen zu geringeren Ausbeuten und dem vermehrten Auftreten unerwünschter Nebenprodukte. Umsetzungen der Verbindungen IIa oder IIb in Schritt (i) gelingen dagegen im Allgemeinen in hohen Ausbeuten ohne nennenswerte Bildung von Nebenprodukten. Es wird vermutet, dass durch die höhere Raumerfüllung der geschützten Aminofunktion in ortho-Position zur Halogenkohlenstoffbindung, in die das katalytisch aktive Palladium zunächst oxidativ inseriert, der Austritt aus der Ligandensphäre des Palladiums im letzten Schritt des katalytischen Prozesses, beschleunigt wird.

Zinkorganylverbindungen der Formel III sowie die Imin- bzw. Amidin-derivatisierten Anilinverbindungen der Formeln IIa und IIb sind allgemein bekannt und können gemäß gängiger Verfahren hergestellt werden. So sind die Zinkorganyle III wie oben beschrieben durch Transmetallierung, vorzugsweise aus den entsprechenden Lithium- und Magnesium-organischen Verbindungen, erhältlich. Die Imine IIa lassen sich vorteilhafterweise durch Umsetzung der entsprechenden Anilinverbindung II mit gegebenenfalls substituiertem Benzaldehyd erzeugen. Zur Herstellung eines Amidins IIb wird die entsprechende Anilinverbindung II vorzugsweise mit einem aktiviertem Derivat des jeweiligen Dialkylformamids, insbesondere einem Acetal des Dialkylformamids, umgesetzt.

Geeignete Palladium-Katalysatoren für die Umsetzung der Verbindungen II mit Verbindungen III in Schritt (i) des erfindungsgemäßen Verfahrens sind palladiumhaltige Verbindungen, worin Palladium eine Oxidationsstufe von 0 oder 2 aufweist.

Beispiele für palladiumhaltige Verbindungen, die eine Oxidationsstufe von 0 aufweisen sind Palladium(0)ligandkomplexe, wie Palladium(0)tetrakis(triphenylphosphin), Palladium(0)tetrakis(diphenylmethylphosphin) oder Palladium(0)-bis-(DIPHOS), oder metallisches Palladium, das gegebenfalls geträgert ist. Metallisches Palladium ist bevorzugt auf einen inerten Träger, wie Aktivkohle, Aluminiumoxid, Bariumsulfat, Bariumcarbonat oder Calciumcarbonat, aufgezogen. Die Umsetzung in Gegenwart von metallischem Palladium erfolgt bevorzugt in Gegenwart geeigneter Komplexliganden.

Beispiele für palladiumhaltige Verbindungen, die eine Oxidationsstufe von 2 aufweisen sind Palladium(II)ligandkomplexe, wie Palladium(II)acetylacetonat oder Verbindungen der Formel PdX₂L₂ worin X für Halogen steht und L insbesondere für einen der im Folgenden genannten Liganden steht, sowie Palladium(II)salze, wie beispielsweise Palladiumacetat oder Palladiumchlorid.

Der Palladium-Katalysator kann in Form eines fertigen Palladium-Komplexes oder als Palladiumverbindung, die unter den Reaktionsbedingungen als Prä-Katalysator zusammen mit geeigneten Liganden die katalytisch aktive Verbindung bildet, eingesetzt werden.

Bei Verwendung von Palladium(II)salzen als Prä-Katalysator erfolgt die Umsetzung bevorzugt in Gegenwart geeigneter Komplexliganden, speziell der im Folgenden genannten Komplexliganden. Dafür infrage kommende Palladium(II)salze sind beispielsweise Palladium(II)chlorid, Bisacetonitrilpalladium(II)chlorid und Palladium(II)acetat. Bevorzugt wird hierzu Palladium(II)chlorid verwendet.

Geeignete Komplexliganden für die Umsetzung in Schritt (i) des erfindungsgemäßen Verfahrens sind beispielsweise mono- oder bidentate Phosphine der im Folgenden gezeigten Formeln VI und VII 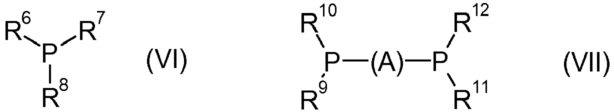 worin R⁶ bis R¹² unabhängig voneinander C₁-C₈-Alkyl, C₅-C₈-Cycloalkyl, Adamantyl, Aryl-C₁-C₂-alkyl, Ferrocenyl oder Aryl, das gegebenenfalls durch C₁-C₄-Alkyl, C₁-C₄-Alkoxy, Fluor oder Chlor substituiert sein kann, stehen und A für Ferrocendiyl oder ein lineares C₂-C₅-Alkandiyl steht, das gegebenenfalls durch C₁-C₈-Alkyl oder C₃-C₆-Cycloalkyl substituiert ist und das gegebenenfalls Teil eines oder zweier mono- oder bicyclischen Ringe ist, welche unsubstituiert oder substituiert sind.

Insbesondere steht A in der Verbindung der Formel VII für C₂-C₄-Alkylen, C₀-C₁-Alkylenferrocenyl, 1,1'-Biphenyl-2,2'-diyl oder 1,1'-Binaphtyl-2,2'-diyl, wobei die vier zuletzt genannten Gruppen gegebenenfalls durch C₁-C₈-Alkyl oder C₁-C₄-Alkoxy substituiert sein können und wobei C₂-C₄-Alkylen zusätzlich einen oder mehrere Substituenten ausgewählt unter C₃-C₇-Cykloalkyl, Aryl oder Benzyl aufweisen kann. In diesem Zusammenhang können 1 bis 4 Kohlenstoffatome des C₂-C₄-Alkylens Teil eins C₃-C₇-Cykloalkylringes sein. Aryl steht hierfür Naphtyl oder gegebenenfalls substituiertes Phenyl. Bevorzugt steht Aryl für Phenyl oder Toluyl, besonders bevorzugt für Phenyl. C₀-C₁-Alkylenferrocenyl steht speziell für Ferrocendiyl, wobei an jedes Cyclopentadien des Ferrocens jeweils eines der beiden Phosphoratome gebunden ist, oder für Methylenferrocenyl, wobei eines der Phosphoratome über die Methylengruppe an ein Cyclopentadien gebunden ist, das zweite Phosphoratom an das selbe Cyclopentadien gebunden ist und die Methylengruppe gegebenenfalls 1 oder 2 weitere Substituenten ausgewählt unter C₁-C₄-Alkyl aufweisen kann.

Für die erfindungsgemäßen Umsetzungen bevorzugte monodentate Komplexliganden der Formel VI sind solche, worin R⁶, R⁷ und R⁸ für gegebenenfalls substituiertes Phenyl stehen, wie beispielsweise Triphenylphosphin (TPP), sowie solche, worin R⁶, R⁷ und R⁸ für C₁-C₆-Alkyl, C₅-C₈-Cycloalkyl oder Adamantyl stehen, wie beispielsweise Di-1-adamantyl-n-butylphosphin, Tri-tert.-butylphosphin (TtBP), Methyl-di-tert.-butylphosphin, Tricyclohexylphosphin und 2-(Dicyclohexylphosphino)-biphenyl. Darüber hinaus können auch Phosphite wie beispielsweise Tris-(2,4-di-tert.-butylphenyl)-phosphit (vergl. A. Zapf et al., Chem. Eur. J. 2000, 6, 1830) verwendet werden.

Gemäß einer bevorzugten Ausführungsform der Erfindung umfaßt der in der Umsetzung in Schritt (i) des erfindungsgemäßen Verfahrens eingesetzte Komplexligand einen bidentaten Komplexliganden der Formel VII. Unter den bidentaten Liganden der Formel VII sind insbesondere Liganden bevorzugt, die der Formel VIII entsprechen: 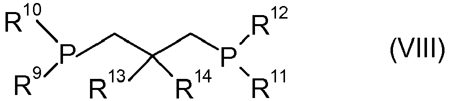 worin R⁹ bis R¹² die oben genannten Bedeutungen haben und vorzugsweise unabhängig voneinander für Phenyl stehen, das gegebenenfalls ein bis drei Substituenten ausgewählt unter Methyl, Methoxy, Fluor und Chlor, trägt. R¹³ und R¹⁴ stehen unabhängig voneinander für Wasserstoff, C₁-C₈-Alkyl oder C₃-C₆-Cycloalkyl, oder R¹³ und R¹⁴ bilden zusammen mit dem Kohlenstoffatom an das sie gebunden sind einen 3- bis 8-gliedrigen Ring, der gegebenenfalls mit C₁-C₆-Alkyl substituiert ist. Vorzugsweise sind R¹³ und R¹⁴ unabhängig voneinander ausgewählt unter Methyl, Ethyl, 1-Methylethyl, n-Butyl, 1-Methylpropyl, 2-Methylpropyl, Pentyl, 1-Methylbutyl, 2-Methylbutyl, 3-Methylbutyl, Hexyl, 1-Methylpentyl, 2-Methylpentyl, 3-Methylpentyl, 4-Methylpentyl, Cyclopropyl, Cyclobutyl, Cyclopentyl oder Cyclohexyl. Beispiele für bevorzugte Verbindungen der Formel (VIII) sind 1,3-Bis-(diphenylphosphanyl)-2-methylpropan, 1,3-Bis-(diphenylphospanyl)-2,2-dimethylpropan, 1,3-Bis-(diphenylphosphanyl)-2-methyl-2-ethylpropan, 1,3-Bis-(diphenylphosphanyl)-2,2-diethylpropan, 1,3-Bis-(diphenylphospanyl)-2-methyl-2-propylpropan, 1,3-Bis-(diphenylphosphanyl)-2-ethyl-2-propypropan, 1,3-Bis-(diphenylphosphanyl)-2,2-dipropylpropan, 1,3-Bis-(diphenylphosphanyl)-2-methyl-2-butylpropan, 1,3-Bis-(diphenylphosphanyl)-2-ethyl-2-butylpropan, 1,3-Bis-(diphenylphosphanyl)-2-propyl-2-butylpropan, 1,3-Bis-(diphenylphosphanyl)-2,2-dibutylpropan, 1,3-Bis-(diphenylphosphanyl)-2-methyl-2-cyclopropylpropan, 1,3-Bis-(diphenylphosphanyl)-2-methyl-2-cyclobutylpropan, 1,3-Bis-(diphenylphosphanyl)-2-methyl-2-cyclopentyl-propan, 1,3-Bis-(diphenylphosphanyl)-2-methyl-2-cyclohexylpropan. Beispiele für besonders bevorzugte Verbindungen der Formel (VIII) sind 1,3-Bis-(diphenylphospanyl)-2,2-dimethylpropan und 1,3-Bis-(diphenylphosphanyl)-2-ethyl-2-butylpropan.

Gemäß einer weiteren bevorzugten Ausführungsform der Erfindung umfassen die in der Umsetzung in Schritt (i) des erfindungsgemäßen Verfahrens als Komplexliganden eingesetzten Liganden wenigstens ein *N*-heterocyclisches Carben, so genannte NHC-Liganden. Dabei handelt es sich insbesondere um reaktive Komplexliganden, die zum Beispiel in G. A. Grasa et al., Organometallics 2002, 21, 2866, beschrieben sind. NHC-Liganden können in situ aus Imidazoliumsalzen, wie z.B. 1,3-Bis-(2,6-diisopropylphenyl)-4,5-H2-imidazoliumchlorid, mit Basen erzeugt und in Gegenwart von Palladium-(0)-Verbindungen, insbesondere des Typs Tris(dibenzylidenaceton)dipalladium(0) oder Bis-(dibenzylidenaceton)palladium(0), oder Palladium-(II)-Salzen wie Palladium-(II)-acetat zu geeigneten Katalysatoren umgesetzt werden. Es ist aber auch möglich, (NHC)-Palladium-(II)-komplexsalze, insbesondere (1,3-Bis-(2,6-diisopropylphenyl)-imidazol-2-yliden)-(3-chlorpyridyl)-palladium(II)-dichlorid, vorab herzustellen und zu isolieren und dann als präformierte Katalysatoren in den erfindungsgemäßen Kreuzkupplungen einzusetzen (vergl. S. P. Nolan, Org. Lett. 2005, 7, 1829 und M. G. Organ, Chem. Eur. J. 2006, 12, 4749).

Für die erfindungsgemäßen Umsetzungen werden als NHC-Liganden vorzugsweise sterisch gehinderte Imidazol-2-yliden-Verbindungen eingesetzt, insbesondere solche der Formel IX, die in den Positionen 1 und 3 des Iminidazolrings sperrige Substituenten R¹⁵ und R¹⁶ tragen. Bevorzugt stehen hier R¹⁵ und R¹⁶ unabhängig voneinander für Aryl oder Hetaryl, die jeweils unsubstituiert sind oder 1, 2, 3 oder 4 Substituenten tragen, wobei die Substituenten vorzugsweise ausgewählt sind unter C₁-C₈-Alkyl und C₃-C₇-Cylcoalkyl. Besonders bevorzugte Substituenten R¹⁵ und R¹⁶ sind Phenylreste, die in den Positionen 2 und 6 vorzugsweise verzweigte C₁-C₆-Alkylreste tragen.

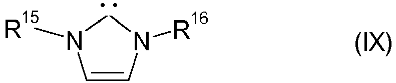

Gemäß einer weiteren bevorzugten Ausführungsform der Erfindung wird für die Umsetzung in Schritt (i) des erfindungsgemäßen Verfahrens ein Palladium-Komplex eingesetzt, der als Komplexliganden wenigstens einen der zuvor genannten NHC-Liganden und gegebenenfalls wenigstens einen weiteren Co-Liganden umfaßt. Solche Co-Liganden sind beispielsweise ausgewählt unter Hetaryl mit zumindest einem Stickstoffatom im Ring, insbesondere unter Pyridyl, das unsubstituiert ist oder 1, 2 oder 3 Substitutenten ausgewählt unter Halogen, C₁-C₆-Alkyl und C₁-C₈-Alkoxy, trägt. Ein konkretes Beispiel für einen solchen Co-Liganden ist 3-Chlorpyridyl.

Gemäß einer besonders bevorzugten Ausführungsform der Erfindung werden für die Umsetzung in Schritt (i) des erfindungsgemäßen Verfahrens Komplexliganden eingesetzt, die ausgewählt sind unter Tri-tert.-butylphosphin, Methyl-di-tert.-butylphosphin, 1,3-Bis-(diphenylphospanyl)-2,2-dimethylpropan, 1,3-Bis-(diphenylphosphanyl)-2-ethyl-2-butylpropan und 1,3-Bis-(2,6-diisopropylphenyl)-imidazol-2-yliden.

Wird eine Palladiumverbindung als Prä-Katalysator zusammen mit geeigneten Liganden in dem erfindungsgemäßen Verfahren eingesetzt, so werden in der Regel 0,5 bis 5 Moläquivalente der vorstehend genannten Komplexliganden mit einem Äquivalent eines Palladium(II)salzes oder einer Palladium(0)verbindung kombiniert. Besonders bevorzugt ist der Einsatz von einem bis drei Moläquivalenten Komplexligand, bezogen auf die Palladiumverbindung.

Der Palladium-Katalysator wird in dem erfindungsgemäßen Verfahren bevorzugt in einer Menge von 0,001 bis 5,0 Mol-%, vorzugsweise von 0,01 bis 1,0 Mol-%, und insbesondere von 0,1 bis 0,5 Mol-%, bezogen auf die eingesetzte Menge der Zinkorganyl-Verbindung III eingesetzt.

Die Reaktionstemperatur des Schrittes (i) wird von mehreren Faktoren bestimmt, wie beispielsweise der Reaktivität der eingesetzten Edukte und der Art des gewählten Palladium-Katalysators, und kann vom Fachmann im Einzelfall ermittelt werden, beispielsweise durch einfache Vorversuche. Im Allgemeinen führt man die Umsetzung in Schritt (i) des erfindungsgemäßen Verfahrens bei einer Temperatur im Bereich von 0 bis 200 °C, vorzugsweise im Bereich von 10 bis 130 °C, insbesondere im Bereich von 25 bis 130 °C und besonders bevorzugt im Bereich von 30 bis 65 °C durch. In Abhängigkeit vom verwendeten Lösungsmittel, von der Reaktionstemperatur und davon ob das Reaktionsgefäß über einen Druckausgleich verfügt, stellt sich während der Reaktion in der Regel ein Druck von 1 bis 6 bar und vorzugsweise von 1 bis 4 bar ein.

Als Reaktionsprodukt der Umsetzung in Schritt (i) des erfindungsgemäßen Verfahrens wird, falls man eine Anilinverbindung II bei der X für NH₂ steht einsetzt, ein 2-Aminobiphenyl der Formel I erhalten. Die Aufarbeitung des erhaltenen Reaktionsgemisches erfolgt in der Regel mit einer wässrigen Säure, d. h. das Reaktionsgemisch wird mit einer wässrigen Lösung einer Säure in Kontakt gebracht. Vorzugsweise werden wässrige Mineralsäuren, insbesondere Salzsäure, in Konzentrationen von in der Regel 1 bis 25 Gew.-% und bevorzugt von 5 bis 15 Gew.-% eingesetzt. Gegebenenfalls wird vor oder nach der Zugabe der wässrigen Säure der als Feststoff anfallende Palladium-Katalysator, beispielsweise durch Filtration, abgetrennt. Aus dem so erhaltenen wasserhaltigen Reaktionsgemisch lässt sich das 2-Aminobiphenyl der Formel I in der Regel durch Extraktion mit einem organischen Lösungsmittel und anschließendem Entfernen des organischen Lösungsmittels isolieren. Im Allgemeinen ist es vorteilhaft vor der Durchführung der Extraktion den pH-Wert des angesäuerten wässrigen Reaktionsgemisches mit einer Base, vorzugsweise einer wässrige Natronlauge, auf einen Wert im basischen Bereich, vorzugsweise im Bereich von 9 bis 13, einzustellen. Das so isolierte 2-Aminobiphenyl I kann anschließend für Verwendungen bereitgehalten oder direkt einer Verwendung zugeführt werden, beispielsweise in einem weiteren Reaktionsschritt eingesetzt werden, oder zuvor weiter aufgereinigt werden. Zur weiteren Aufreinigung kann eine oder mehrere dem Fachmann bekannten Verfahren, wie beispielsweise Umkristallisation, Destillation, Sublimation Zonenschmelzen, Schmelzkritallisation oder Chromatographie verwendet werden.

Verwendet man hingegen in Schritt (i) des erfindungsgemäßen Verfahrens eine Anilinverbindung II bei der X für X¹ oder X² steht, so wird eine Biphenylverbindung la beziehungsweise Ib als Reaktionsprodukt erhalten, welches entweder mit oder ohne vorherige Aufarbeitung im Schritt (ii) des erfindungsgemäßen Verfahrens eingesetzt werden kann. Die Aufarbeitung erfolgt üblicherweise entweder wässrig oder nicht-wässrig. Zur wässrigen Aufarbeitung wird das Reaktionsgemisch in der Regel mit einer gegebenenfalls angesäuerten wässrigen Lösung in Kontakt gebracht. Bei der angesäuerten wässrigen Lösung handelt es sich vorzugsweise um eine verdünnte wässrige Lösung einer mineralischen Säure, insbesondere Salzsäure, mit einer Konzentration von 0,001 bis 5 Gew.-% und insbesondere von 0,1 bis 3 Gew.-%. Gegebenenfalls wird vor oder nach der Zugabe der wässrigen Lösung der als Feststoff anfallende Palladium-Katalysator, beispielsweise durch Filtration, abgetrennt. Aus dem so erhaltenen wasserhaltigen Reaktionsgemisch lässt sich die Biphenylverbindung der Formel Ia oder Ib in der Regel durch Extraktion mit einem organischen Lösungsmittel und anschließendem Entfernen des organischen Lösungsmittels isolieren. Zur nicht-wässrigen Aufarbeitung wird das Reaktionsgemisch in der Regel, nachdem es gegebenenfalls beispielweise mit Aktivkohle behandelt wurde, filtriert, zum Beispiel über Kieselgur, und das Produkt durch Entfernen des Lösungsmittels aus dem Filtrat isoliert. Die so im Anschluss an eine wässrige oder nicht-wässrige Aufarbeitung erhaltene Biphenylverbindung Ia oder Ib kann direkt in Schritt (ii) des erfindungsgemäßen Verfahrens eingesetzt werden oder anderen Verwendungen zugeführt werden. Alternativ kann sie zu einer weiteren Verwendung bereitgehalten oder zunächst, anhand von dem Fachmann bekannten Verfahren, weiter aufgereinigt werden.

Die Verbindungen Ia und Ib sind neu und ebenfalls Gegenstand der vorliegenden Erfindung: 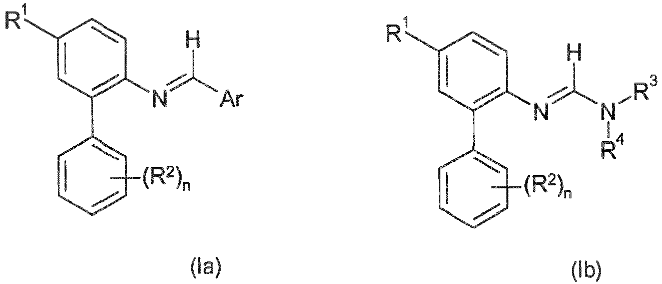 wobei
- n: für 1, 2 oder 3 steht,
- Ar: für Phenyl steht, das gegebenenfalls 1, 2, oder 3 Substituenten trägt, die ausgewählt sind unter C₁-C₄-Alkyl und C₁-C₄-Alkoxy,
- R¹: für Wasserstoff, Cyano oder Fluor steht,
- jedes R²: im Falle der Verbindungen la jeweils unabhängig voneinander ausgewählt ist unter Cyano, Fluor, C₁-C₄-Alkyl, C₁-C₄-Fluoralkyl, C₁-C₄-Fluoralkoxy, C₁-C₄-Alkylthio und C₁-C₄-Fluoralkylthio,
- jedes R²: im Falle der Verbindungen Ib jeweils unabhängig voneinander ausgewählt ist unter Cyano, Fluor, C₁-C₄-Fluoralkyl, C₁-C₄-Alkoxy, C₁-C₄-Fluoralkoxy, C₁-C₄-Alkylthio und C₁-C₄-Fluoralkylthio, und
- R³ und R⁴: unabhängig voneinander für C₁-C₆-Alkyl stehen.

In bevorzugten Verbindungen der Formel la steht jedes R² jeweils unabhängig voneinander für Fluor, C₁-C₄-Alkyl oder C₁-C₄-Fluoralkyl. In bevorzugten Verbindungen der Formel Ib steht jedes R² jeweils unabhängig voneinander für Fluor oder C₁-C₄-Fluoralkyl. In besonders bevorzugten Verbindungen der Formel Ia oder Ib steht jedes R² jeweils für Fluor. R¹ steht insbesondere für Wasserstoff oder Fluor. Ar steht insbesondere für Phenyl. R³ und R⁴ stehen insbesondere jeweils für Methyl oder Ethyl. Eine ganz besonders bevorzugte Verbindung der Formel Ia ist 2-Phenylmethylidenamino-(3',4',5'-trifluoro)-biphenyl und eine ganz besonders bevorzugte Verbindung der Formel Ib ist *N,N*-Dimethyl-*N*'-(3',4',5'-trifluoro-biphenyl-2-yl)-formamidin.

In Schritt (ii) des erfindungsgemäßen Verfahrens zur Herstellung substituierter 2-Aminobiphenyle I wird das aus Schritt (i) erhaltene Reaktionsprodukt in der Regel unter hydrolytischen Bedingungen umgesetzt, um vorliegende Imim- oder Amidingruppen in Aminogruppen zu überführen. Üblicherweise wird Schritt (ii) unter Temperaturkontrolle und entsprechend einer bevorzugen Ausführungsform in einem Reaktionsgefäß mit Rückflußkühler und Heizvorrichtung sowie gegebenenfalls einer Destillationsvorrichtung durchgeführt.

Handelt es sich bei dem aus Schritt (i) erhaltenen Reaktionsprodukt um eine Verbindung la, so erfolgt die Hydrolyse in der Regel sauer, wie beispielsweise von T. Nozoe et al., Bull. Chem. Soc. Jpn. 1989, 62, 2307, beschrieben. Gemäß einer bevorzugten Ausführungsform werden für die Hydrolyse wässrige Mineralsäuren, insbesondere Salzsäure, in der Regel in einer Konzentration von vorzugsweise 1 bis 20 Gew.-% und besonders bevorzugt 2 bis 10 Gew.-% eingesetzt. Nach der Zugabe der Säure wird typischerweise 30 Minuten bis 10 Stunden und insbesondere 1 bis 5 Stunden auf eine Temperatur von 25 bis 100 °C und insbesondere von 50 bis 90 °C erhitzt. Falls im Anschluss an Schritt (i) das Lösungsmittel entfernt worden ist, wird gegebenenfalls zuvor in einem vorzugsweise polaren organischen Lösungsmittel aufgenommen. Das im Anschluss an die hydrolytische Umsetzung der Verbindung la erhaltene Reaktionsgemisch wird mit dem Fachmann bekannten Verfahren aufgearbeitet. Beispielsweise kann, falls es sich bei dem Rest Ar um Phenyl handelt, zunächst das freigesetzte Benzaldehyd im Azeotrop mit Wasser abdestilliert werden und der wässrige Rückstand, gegebenenfalls nach Einstellen des pH-Werts, mit einem organischen Lösungsmittel extrahiert werden.

Handelt es sich bei dem aus Schritt (i) erhaltenen Reaktionsprodukt um eine Verbindung Ib, so erfolgt die Hydrolyse entweder durch Zugabe einer Säure oder einer Base. Die basische Hydrolyse kann beispielsweise in Analogie zu der von A. I. Meyers et al. Tetrahedron Lett. 1990, 31, 4723, beschriebenen Umsetzung durchgeführt werden. Zur sauren Hydrolyse werden vorzugsweise wässrige Mineralsäuren, insbesondere Schwefelsäure, in der Regel als 0,5 bis 5 molare und bevorzugt als 1 bis 3 molare wässrige Lösung eingesetzt. Nach der Zugabe der Säure wird bis zur vollständigen oder annähernd vollständigen Umsetzung der Verbindung 1 b auf eine Temperatur von 25 bis 100 °C und insbesondere von 50 bis 90 °C erhitzt. Falls im Anschluss an Schritt (i) das Lösungsmittel entfernt worden ist, wird gegebenenfalls zuvor in einem vorzugsweise polaren organischen Lösungsmittel, wie beispielsweise Butanol, aufgenommen. Das im Anschluss an die hydrolytische Umsetzung der Verbindung la erhaltene Reaktionsgemisch wird mit dem Fachmann bekannten Verfahren aufgearbeitet. Beispielsweise kann zunächst, falls es sich bei den Resten R³ und R⁴ beispielsweise jeweils um Methyl handelt, das Hydrolyseprodukt der Formel I durch Extraktion mit einem wasserunlöslichen organischen Lösungsmittel von dem freigesetzten Dimethylformamid abgetrennt werden. Dabei kann es vorteilhaft sein, speziell bei Verwendung von mit Wasser mischbaren Lösungsmitteln in den Schritten (i) und/oder (ii), das Lösungsmittel vor der Extraktion wenigstens teilweise zu entfernen, beispielsweise destillativ.

Alternativ kann in Schritt (ii) die Überführung der Amidingruppe einer Verbindung Ib in die Aminofunktion auch durch Hydrierung erfolgen, beispielsweise mittels des von L. Lebeau et al., J. Org. Chem. 1999, 64, 991, beschriebenen Verfahrens.

Die nach der Aufarbeitung der Hydrolyse einer Verbindung la oder Ib gemäß Schritt (ii) erhaltenen Rohprodukte des substituierten 2-Aminobiphenyls I können zur weiteren Aufreinigung einem oder mehreren dem Fachmann bekannten Verfahren, wie beispielsweise Umkristallisation, Destillation, Sublimation, Zonenschmelzen, Schmelzkritallisation oder Chromatographie unterzogen werden.

Gegenstand der Erfindung ist weiterhin ein Verfahren zur Herstellung von Verbindungen der Formel IV, 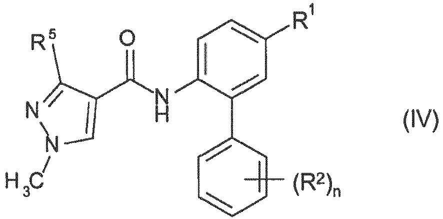 wobei
- n, R¹ und R²: die zuvor für die Verbindungen der allgemeinen Formel I genannten Bedeutungen haben, und
- R⁵: für Methyl oder Halomethyl steht,
umfassend die Bereitstellung des 2-Aminobiphenyls der Formel I durch das erfindungsgemäße Verfahren und anschließende *N*-Acylierung des 2-Aminobiphenyls der Formel I mit einer Verbindung der Formel V, 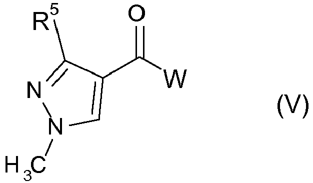 wobei R⁵ die oben genannte Bedeutung hat und W eine Abgangsgruppe bedeutet, unter Erhalt einer Verbindung der Formel IV.

Bezüglich geeigneter und bevorzugter Verbindungen I wird vollständig auf das zuvor Gesagte Bezug genommen.

Die Überführung einer Verbindung I in ein Pyrazolcarboxamid der Formel IV erfolgt gemäß üblichen Verfahren des Standes der Technik zur Amidbildung.

In der Regel wird zur erfindungsgemäßen *N*-Acetylierung eines Aminobiphenyls I als Reagenz V eine Carbonsäure oder ein zur Amidbildung befähigtes Derivat einer Carbonsäure, wie etwa ein Säurehalogenid, Säureanhydrid oder Ester, eingesetzt. Dementsprechend handelt es sich bei der Abgangsgruppe W üblicherweise um Hydroxy, Halogenid, insbesondere Chlorid oder Bromid, einen Rest -OR⁷ oder einen Rest -O-CO-R⁸, wobei die Bedeutungen der Substituenten R⁷ und R⁸ im Folgenden erläutert werden.

Wird das Reagenz V als Carbonsäure (W = OH) eingesetzt, so kann die Reaktion in Gegenwart eines Kopplungsreagenzes durchgeführt werden. Geeignete Kopplungsreagenzien (Aktivatoren) sind dem Fachmann bekannt und sind beispielsweise ausgewählt unter Carbodiimiden, wie DCC (Dicyclohexylcarbodiimid) und DCl (Diisopropylcarbodiimid), Benzotriazolderivaten, wie HBTU ((O-Benzotriazol-1-yl)-N,N',N'-Tetramethyluroniumhexafluorophosphat) und HCTU (1 H-Benzotriazolium-1-[bis(dimethylamino)methylen]-5-chlor-tetraflouroborat) und Phosphonium-Aktivatoren, wie BOP ((Benzotriazol-1-yloxy)-tris(dimethylamino)phosphoniumhexafluorophosphat), Py-BOP ((Benzotriatzol-1-yloxy)-tripyrrolidinphosphoniumhexafluorophosphat) und Py-BrOP (Bromtripyrrolidinphosphoniumhexafluorophosphat). Im Allgemeinen wird der Aktivator im Überschuss eingesetzt. Die Benzotriazol- und Phosphonium-Kopplungsreagenzien werden in der Regel in einem basischen Medium eingesetzt.

Geeignete Derivate der Carbonsäure Y-COOH, wobei Y für den Pyrazolring der Verbindung V steht, sind alle Derivate, die mit dem 2-Aminobiphenyl I zu dem Amid IV reagieren können, wie beispielsweise Ester Y-C(O)-OR⁷ (W = OR⁷), Säurehalogenide Y-C(O)X, worin X für ein Halogenatom steht (W = Halogen), oder Säureanhydride Y-CO-O-OC-R⁸ (W = -O-CO-R⁸)

Bei dem Säureanhydrid Y-CO-O-OC-R⁸ handelt es sich entweder um ein symmetrisches Anhydrid Y-CO-O-OC-Y (R⁸ = Y), oder um ein asymmetrisches Anhydrid, worin -O-OC-R⁸ für eine Gruppe steht, die durch das in die Reaktion eingesetzte 2-Aminobiphenyl I leicht verdrängt werden kann. Geeignete Säurederivate, mit der die Carbonsäure Y-COOH geeignete gemischte Anhydride bilden kann, sind beispielsweise die Ester von Chlorameisensäure, z. B. Isopropylchlorformiat und Isobutylchlorformiat, oder von Chloressigsäure.

Geeignete Ester Y-COOR⁷ leiten sich vorzugsweise von C₁-C₄-Alkanolen R⁷OH ab, worin R⁷ für C₁-C₄-Alkyl steht, wie Methanol, Ethanol, Propanol, Isopropanol, n-Butanol, Butan-2-ol, Isobutanol und tert-Butanol, wobei die Methyl- und Ethylester (R⁷ = Methyl oder Ethyl) bevorzugt sind. Geeignete Ester können sich auch von C₂-C₆-Polyolen, wie Glykol, Glycerin, Trimethylolpropan, Erythritol, Pentaerythritol und Sorbitol, ableiten, wobei der Glycerinester bevorzugt ist. Wenn Polyol-Ester eingesetzt werden, so können gemischte Ester, d. h. Ester mit verschiedenen Resten R⁷, verwendet werden.

Alternativ handelt es sich bei dem Ester Y-COOR⁷ um einen sogenannten Aktivester, den man formal durch die Umsetzung der Säure Y-COOH mit einem Aktivesterbildenden Alkohol, wie p-Nitrophenol, *N*-Hydroxybenzotriazol (HOBt), N-Hydroxysuccinimid oder OPfp (Pentaflourphenol) erhält.

Alternativ kann das zur *N*-Acylierung eingesetzte Reagenz V über eine andere gebräuchliche Abgangsgruppe W verfügen, beispielsweise Thiophenyl oder Imidazolyl.

Die erfindungsgemäßen *N*-Acylierungen mit den zuvor beschriebenen Reagenzien der Formel V können analog bekannter Verfahren durchgeführt werden.

Vorzugsweise werden für die *N*-Acylierung von Verbindungen I Carbonsäurehalogenide V eingesetzt, insbesondere solche, bei denen die Abgangsgruppe W für Chlor oder Brom, und besonders bevorzugt für Chlor steht. Dazu werden vorzugsweise 0,5 bis 4 Mol und insbesondere 1 bis 2 Mol des Säurechlorids pro 1 Mol der Verbindung I verwendet.

Üblicherweise wird die *N*-Acylierung eines Aminobiphenyls I mit einem Säurechlorid V in Gegenwart einer Base, wie etwa Triethylamin, durchgeführt, wobei in der Regel 0.5 bis 10 Mol, insbesondere 1 bis 4 Mol der Base pro 1 Mol des Säurechlorids eingesetzt werden.

Häufig wird man zur Herstellung einer Verbindung der Formel IV die entsprechende Verbindung I zusammen mit der Base vorzugsweise in einem Lösungsmittel vorlegen und bei einer Temperatur im Bereich von etwa -30°C bis 50°C, insbesondere von 0°C bis 25°C, das Säurechlorid, gegebenenfalls in einem Lösungsmittel gelöst, schrittweise zugeben. Üblicherweise lässt man anschließend bei erhöhter Temperatur, etwa im Bereich von 0°C bis 150°C, insbesondere von 15°C bis 80°C, weiterreagieren.

Die Acylierung kann jedoch auch in Abwesenheit einer Base durchgeführt werden. Hierzu wird die Acylierung in einem Zweiphasensystem durchgeführt. Dabei ist eine der Phasen wässrig und die zweite Phase basiert auf wenigstens einem mit Wasser im Wesentlichen nicht mischbaren organischen Lösungsmittel. Geeignete wässrige Lösungsmittel und geeignete mit Wasser im Wesentlichen nicht mischbare organische Lösungsmittel sind in der WO 03/37868 beschrieben. In dieser Literaturstelle sind auch weitere geeignete Reaktionsbedingungen für Acylierungsverfahren in Abwesenheit von Basen allgemein beschrieben.

Die Aufarbeitung der bei den Umsetzungen zur erfindungsgemäßen *N*-Acylierung erhaltenen Reaktionsgemische und die Isolierung der Verbindung der Formel IV erfolgt in üblicher Weise, beispielsweise durch eine wässrige, extraktive Aufarbeitung, durch Entfernen des Lösungsmittels, z. B. unter vermindertem Druck, oder durch eine Kombination dieser Maßnahmen. Eine weitere Reinigung kann beispielsweise durch Kristallisation, Destillation oder durch Chromatographie erfolgen.

Durch die erfindungsgemäßen Verfahren lassen sich zum Einen die 2-Aminobiphenyle I mit geringem Aufwand, in guten bis sehr guten Ausbeuten und mit hohen Selektivitäten herstellen, und sind zum Anderen die davon abgeleiteten Carbonsäureamide IV leicht und in der Regel quantitativ zugänglich.

### Beispiele

### A. Herstellung der Zinkorganylverbindungen der Formel III sowie der analogen Grignard-Verbindungen

Die nachfolgenden Beispiele sollen exemplarisch zeigen, wie die in den erfindungsgemäßen Verfahren eingesetzten Zinkorganylverbindungen sowie die analogen Magnesiumorganylverbindungen (Grignard-Verbindungen) hergestellt werden.

### A.1 3,4,5-Trifluorphenylmagnesiumbromid

In einem mit Stickstoff oder Argon inertisierten Reaktor legte man zunächst Magnesiumspäne (83,2 g; 3,42 mol) vor und gab anschließend trockenes, unstabilisiertes Tetrahydrofuran (THF, 1646,2 g) zu. Unter Rühren wurde zu dieser Suspension bei einer Temperatur von 25°C 3,4,5-Trifluorbrombenzol (30 g; 0,14 mol) zugetropft und das Anspringen der Reaktion abgewartet, das sich durch eine spontane Temperaturerhöhung auf ca. 32°C bemerkbar machte. Anschließend dosierte man weiteres 3,4,5-Trifluorbrombenzol (571,9 g; 2,71 mol) innerhalb von 5 h bei einer Temperatur von 25 bis 35°C zu. Zur Vervollständigung der Reaktion wurde 2 h bei 25 bis 30°C nachgerührt. Danach wurde das Reaktionsgemisch filtriert, das abfiltrierte überschüssige Magnesium mit wenig THF gewaschen und die Waschlösung mit dem Filtrat vereinigt. Der Gehalt an 3,4,5-Trifluorphenylmagnesiumbromid einer so hergestellten Lösung in THF wurde ausgehend vom vollständigen Umsatz des eingesetzten 3,4,5-Trifluorbrombenzols auf 1,1 bis 1,2 mmol/g Lösung kalkuliert.

### A.2 3,4,5-Trifluorphenylzinkbromid

Eine etwa 1 M Lösung von 3,4,5-Trifluorphenylzinkbromid in THF erhielt man mittels einer zur Herstellung des 3,4,5-Trifluorphenylmagnesiumbromids analogen Umsetzung, bei der die Magnesiumspäne durch Rieke-Zink ersetzt wurden.

### B Herstellung der N-derivatisierten 2-Anilinverbindungen der Formeln Ila und Ilb

Die nachfolgenden Beispiele sollen exemplarisch zeigen, wie die in den erfindungsgemäßen Verfahren eingesetzten Imin- bzw. Amidin-Derivate der Anilinverbindung II hergestellt werden.

### B.1 Herstellung von 1-Phenylmethylidenamino-2-chlor-benzol

In Ethanol (832 g) löste man 98%iges 2-Chloranilin (781 g; 6 mol) und 98%igen Benzaldehyd (659,6 g; 6 mol) und kochte die Lösung 8 h unter Rückfluss (Heizvorrichtung auf 90°C eingestellt). Anschließend wurde die Reaktionslösung am Rotationsverdampfer bei 85°C und 20 mbar eingeengt und dann über eine Destillationsbrücke fraktioniert destilliert. Die Hauptfraktion ging bei 142°C und 1,3 mbar über. Es wurden 1048 g des Produkts in 99%iger Reinheit erhalten.
EI-MS [m/z]: 215 [M]⁺;
¹H-NMR (500 MHz, CDCl₃): δ = 7,0 (d, 1 H); 7,1 (t, 1 H); 7,23 (dd, 1 H); 7,4 (d, 1 H); 7,45-7,5 (m, 3H); 7,93 (d, 2H); 8,34 (s, 1 H) ppm;
¹³C-NMR (125 MHz, CDCl₃): δ = 118,9; 126,3, 127,8; 128,8; 129,1; 129,4; 130,1; 131,8; 35,9; 149,6; 162,1 ppm.

### B.2 Herstellung von N,N-Dimethyl-N'-(2-chlor-phenyl)-formamidin

In Toluol (300 g) löste man 98%iges 2-Chloranilin (195 g; 1,5 mol) und 98%iges Dimethylformamid-dimethylacetal (228,2 g; 1,8 mol) und erhitzte die Lösung 4 h unter Rückfluss. Während dieser Zeit fiel die Innentemperatur von 96 auf 77 °C ab. Danach wurden 15 g Destillat abgenommen und die Innentemperatur stieg währenddessen wieder auf 84°C an. Schließlich engte man die Reaktionslösung am Rotationsverdampfer bei 90°C und 5 mbar ein und fraktionierte das erhaltende braune Öl (270,5 g) mittels Kolonnendestillation. Die Hauptfraktion ging bei 120°C und 1,4 mbar über und enthielt das Produkt in einer Reinheit von > 99% (bestimmt anhand Flächenprozente des GC-Spektrums).
EI-MS [m/z]: 182 [M]⁺;
¹H-NMR (500 MHz, DMSO): δ = 2,9 (s, 6H); 6,85-6,95 (m, 2H); 7,1-7,16 (m, 1H); 7,34 (d, 1 H); 7,58 (s, 1 H) ppm;
¹³C-NMR (125 MHz, CDCl₃): δ = 33,9; 120,8; 122,5; 127,2; 127,4; 129,4; 149,1; 153,6 ppm.

### C Herstellung von kernsubstituierten 2-Aminobiphenylen der Formel I aus Anilinverbindungen der Formel II mit freier Aminogruppe

Die nachfolgenden Beispiele sollen exemplarisch zeigen, wie mittels des erfindungsgemäßen Verfahrens ausgehend von den 2-Halogenanilinen der Formel II die substituierten 2-Aminobiphenyle der Formel I hergestellt werden. In beiden Beispielen wird das Zinkorganyl in-situ aus der entsprechenden Grignard-Verbindung erzeugt.

### C.1 Herstellung von 3,4,5-Trifluor-2'-amino-biphenyl aus 2-Bromanilin (nicht erfindungsgemäß)

In einem 100 ml Glaskoben legte man Zinkchlorid (4,8 mmol) gelöst in Toluol (0,5 M Lösung) vor und gab dazu eine Lösung von 3,4,5-Trifluorphenylmagnesiumbromid (4,5 mmol; 1,15 mmol pro g Lösung) in THF bei einer Temperatur von 25 °C. Nach 20 min Rühren wurde *N*-Methylpyrrolidon (6,8 g) zu der Reaktionslösung gegeben. Weitere 5 min später gab man Tri-tert.-butylphophonium tetrafluoroborat (18,2 mg),
Bis(dibenzylidenaceton)palladium-(0) (18,2 mg) und 2-Bromanilin (0,54 g; 3 mmol) zu. Die Reaktionslösung wurde anschließend für 5 h bei einer Temperatur von 25 °C gerührt. Danach gab man das Reaktionsgemisch auf eine 10 Gew.-%ige Salzsäure und, nachdem der pH-Wert mit Natronlauge auf 12 eingestellt worden war, extrahierte man mit Diethylether.
Die gaschromatographische Analyse der organische Phase ergab, dass das Hauptprodukt, 3,4,5-Trifluor-2'-amino-biphenyl zu dem Edukt 2-Bromanilin sowie den Nebenprodukten 1,2,3-Trifluorbenzol und 3,4,5,3',4',5'-Hexafluorbiphenyl in einem Verhältnis von 44 : 3 : 39 : 3 vorlag. Unter der Voraussetzung, dass aus der eingesetzten Grignard-Verbindung nur das Hauptprodukt und die oben genannten zwei Nebenprodukte enstanden sind, ergibt sich daraus eine Ausbeute von 55% bezüglich des 2-Bromanilins.

### C.2 Herstellung von 3,4,5-Trifluor-2'-amino-biphenyl aus 2-Chloranilin

In einem 100 ml Glaskoben gab man zu festem Zinkchlorid (0,9 g; 6,4 mmol) bei einer Temperatur von 25 °C eine Lösung von 3,4,5-Trifluorphenylmagnesiumbromid (4,5 mmol; 1,15 mmol pro g Lösung) in THF. Nach 30 min Rühren wurden zu der Reaktionslösung 12,7 ml *N*-Methylpyrrolidon gegeben. Weitere 15 min später gab man (1,3-Bis-(2,6-diisopropylphenyl)-imidazol-2-yliden)(3-chlorpyridyl)palladium(II)-dichlorid (27 mg) und nach weiteren 15 min 2-Chloranilin (0,51 g; 4 mmol) zu. Die so erhaltene Reaktionslösung wurde 2 h bei einer Temperatur von 25°C gerührt und anschließend 4 h unter Rückfluss erhitzt. Danach hydrolysierte man eine gewogene Probe des Reaktionsgemischs in einem Messkolben mit 1 ml 10%ige Salzsäure und füllte mit Acetonitril und Wasser bis zur Markierung auf. Die per quantitativer HPLC durchgeführte Analyse der so erhaltenen Messlösung ergab, dass die Reaktionsmischung 2,6 mmol 3,4,5-Trifluor-2'-amino-biphenyl enthielt. Dies entspricht einer Ausbeute von 65% bezogen auf 2-Chloranilin.

### D Herstellung von N-derivatisierten 2-Aminobiphenylen der Formeln Ia und Ib aus Anilinderivaten der Formeln Ila und Ilb und anschließende Hydrolyse zu den 2-Aminobiphenylen mit freier Aminogruppe

Die nachfolgenden Beispiele sollen exemplarisch zeigen, wie mittels des erfindungsgemäßen Verfahrens ausgehend von den N-derivatisierten 2-Halogenanilinen der Formeln Ila und Ilb die N-derivatisierten 2-Aminobiphenyle der Formeln la und Ib hergestellt und diese gegebenenfalls dann in die entsprechenden 2-Aminobiphenyle der Formel I hydrolytisch überführt werden.

### D.1 Herstellung von 2-Phenylmethylidenamino-3',4',5'-trifluorbiphenyl aus Phenylmethylidenamino-2-chlor-benzol

In einem 750-ml-Reaktor erwärmte man eine Mischung aus Zinkchlorid (20 g; 0,147 mol) und THF (76 g) auf eine Temperatur von 30 °C. Anschließend wurde eine Lösung von 3,4,5-Trifluorphenylmagnesiumbromid (0,133 mol) in THF (1,15 mmol pro g Lösung) zugegeben. Nach 10-minütigem Rühren gab man (1,3-Bis-(2,6-diisopropylphenyl)-imidazol-2-yliden)(3-chlorpyridyl)palladium(II)-dichlorid (462 mg) bei einer Temperatur von 25 °C zu. Weitere 5 min später wurden zu der Reaktionsmischung *N*-Methylpyrrolidon (30 g) und nach weiteren 10 min 99-%iges Phenylmethylidenamino-2-chlor-benzol (29 g; 0,133 mol) zugeben. Danach erwärmte man auf 50 °C und rührte 6 h bei dieser Temperatur. Zur Aufarbeitung wurden Wasser (300 g), konz. Salzsäure (22 g) sowie Diethylether (300 g) zugegeben und im Schütteltrichter durchmischt. Anschließend trennte man die organische Phase ab und extrahierte die wässrige Phase nochmals mit Diethylether (300 g). Die vereinigten organischen Phasen wurden am Rotationsverdampfer zu einem dunklen, schnell durchkristallisierendem Öl (81,3 g) eingedampft.
Die gaschromatographische Analyse ergab, dass zwei Produkte in einem Verhältnis von 81 : 19 vorlagen, bei denen es sich um das Iminderivat 2-Phenylmethylidenamino-3',4',5'-trifluorbiphenyl und um das daraus entstandene Hydrolyseprodukt 3,4,5-Trifluor-2'-amino-biphenyl handelte. Die Identität des intensiveren Signals der GC-Analyse wurde durch unabhängige Synthese des Iminderivats aus 3,4,5-Trifluor-2'-aminobiphenyl und Benzaldehyd bestätigt.
Da unter den Bedingungen der quantitativen HPLC-Analyse das Iminderivat komplett zu 3,4,5-Trifluor-2'-amino-biphenyl hydrolysiert wurde, entsprach die Gesamtausbeute an Biphenylkupplungsprodukt dem für das 3,4,5-Trifluor-2'-amino-biphenyl per quantitativer HPLC ermittelten Wert von 123 mmol. Dies entspricht einer Ausbeute von 92% bezüglich des eingesetzten Phenylmethylidenamino-2-chlor-benzols.
EI-MS [m/z]: 311 [M]⁺;
¹H-NMR (500 MHz, CDCl₃): δ = 7,05 (d, 1 H); 7,1-7,2 (m, 2H); 7,25-7,3 (m, 1 H); 7,32-7,5 (m, 5H); 7,76-7,82 (d, 2H); 8,45 (s, 1 H) ppm;
¹³C-NMR (125 MHz, CDCl₃): δ = 114,25; 118,95; 126,36; 128,90; 128,94; 129,54; 129,88; 131,66; 132,62; 135,65; 136,14; 138,89; 149,49; 150,62; 160,64 ppm.

### D.2 Herstellung von 3,4,5-Trifluor-2'-aminobiphenyl aus Phenylmethylidenamino-2-chlor-benzol

In einem 1 L-Reaktor erwärmte man eine Mischung aus Zinkchlorid (20 g; 0,147 mol) und THF (29 g) auf eine Temperatur von 30 °C. Anschließend wurde eine Lösung von 3,4,5-Trifluorphenylmagnesiumbromid (0,133 mol) in THF (1,15 mmol pro g Lösung) zugegeben. Nach 10-minütigem Rühren gab man (1,3-Bis-(2,6-diisopropylphenyl)-imidazol-2-yliden)(3-chlorpyridyl)palladium(II)-dichlorid (462 mg) bei einer Temperatur von 25 °C zu. Weitere 5 min später wurden zu der Reaktionsmischung *N-*Methylpyrrolidon (30 g) und nach weiteren 10 min 99-%iges Phenylmethylidenamino-2-chlor-benzol (29 g; 0,133 mol) zugegeben. Danach erwärmte man auf 50 °C und rührte 6 h bei dieser Temperatur. Zur Aufarbeitung wurden 3,4 g Aktivkohle zugegeben, die erhaltene Suspension 1 h gerührt und dann über Kieselgur filtriert. Man spülte den Filterkuchen mit THF (200 g) nach und konzentrierte anschließend das Filtrat am Rotationsverdampfer bei einer Temperatur von 50°C in einem Vakuum von bis zu 5 mbar auf. Zu dem verbleibenden Rückstand wurde Wasser (200 g) und konz. Schwefelsäure (28 g) gegeben. Mann rührte die Mischung 3 h bei einer Temperatur von 80 °C und destillierte den freigesetzten Benzaldehyd im Azeotrop mit Wasser ab. Anschließend wurde mit Natronlauge auf einen pH-Wert von 2,8 eingestellt und Toluol (200 g) zugegeben. Nach der Phasentrennung extrahierte man die wässrige Phase nochmals mit Toluol (200 g) wusch die vereinigten organischen Phasen mit Wasser (100 g) und konzentrierte sie am Rotationsverdampfer bei 80°C und 5 mbar zu einem dunklen Öl (26,7 g) auf, das beim Stehenlassen kristallisierte.
Die Analyse per quantitativer HPLC ergab, dass 86,4 Gew.-% des entstandenen Öls 3,4,5-Trifluor-2'-amino-biphenyl ausmachten, was einer Ausbeute von 78 % entspricht.

### D.3 Herstellung von N,N-Dimethyl-N'-(3',4',5'-trifluoro-biphenyl-2-yl)-formamidin und Hydrolyse zum 3,4,5-Trifluor-2'-amino-biphenyl

In einem 1 L Reaktor legte man eine Lösung (132,2 g; 1 M) von 3,4,5-Trifluorphenylzinkbromid in THF vor und verdünnte mit THF (104,9 g). Danach wurden (1,3-Bis-(2,6-diisopropylphenyl)-imidazol-2-yliden)(3-chlorpyridylpalladium(II)-dichlorid (461,8 mg) bei einer Temperatur von 25°C zugegeben. Weitere 5 min später wurde *N-*Methylpyrrolidon (29,9 g) und nach weiteren 10 min *N,N*-Dimethyl-*N*'-(2-chlor-phenyl)-formamidin (24,4 g; 0,133 mol) zu der Mischung gegeben. Danach erwärmte man auf 50 °C und rührte 6 h bei dieser Temperatur. Nach Stehenlassen über Nacht bei einer Temperatur von 25 °C wurde noch 1 h unter Rückfluss erhitzt und wieder auf 25°C abgekühlt. Zur Aufarbeitung gab man 2,5 g Aktivkohle zu, ließ über Nacht Rühren und filtrierte über Kieselgur. Der Filterkuchen wurde mit 200 g THF nachgewaschen.
Der Anteil des Produkts *N,N*-Dimethyl-*N*'-(3',4',5'-trifluoro-biphenyl-2-yl)-formamidin in dem vereinigten Filtrat (387,8 g) betrug laut Analyse mittels quantitativer HPLC 8,4 Gew.-%, was einer Ausbeute von 89% entspricht.
Die Identität des im Filtrat enthaltenen *N,N*-Dimethyl-*N*'-(3',4',5'-trifluoro-biphenyl-2-yl)-formamidin wurde durch unabhängige Synthese aus 3,4,5-Trifluor-2'-amino-biphenyl und Dimethoxymethyl-dimethylamin bestätigt.
EI-MS [m/z]: 278 [M]⁺;
¹H-NMR (500 MHz, CDCl₃): δ = 2,88 (s, 3H); 3,0 (s, 3H); 6,95 (d, 1 H); 7,02 (t, 1 H); 7,2-7,3 (m, 2H); 7,4-7,5 (m, 2H); 7,6 (s, 1 H) ppm;
¹³C-NMR (125 MHz, CDCl₃): δ = 33,71; 39,21; 114,01; 114,25; 119,67; 122,15; 129,87; 130,65; 137,17; 137,41; 149,62; 149,27; 153,04.

Zur hydrolytischen Umsetzung löste man das Rohprodukt in n-Butanol und erhitzte nach Zugabe von wäßriger Schwefelsäure (2 M) unter Rückfluss. Das Produkt 3,4,5-Trifluor-2'-amino-biphenyl wurde als Lösung in n-Butanol erhalten.

## Patentansprüche

1. Verfahren zur Herstellung substituierter 2-Aminobiphenyle der Formel I, 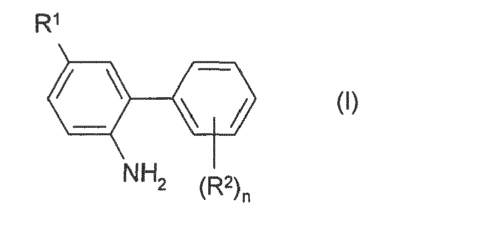 worin
n für 0, 1, 2 oder 3 steht,
R¹ für Wasserstoff, Cyano oder Fluor steht, und
jedes R² jeweils unabhängig voneinander ausgewählt ist unter Cyano, Fluor, C₁-C₄-Alkyl, C₁-C₄-Fluoralkyl, C₁-C₄-Alkoxy, C₁-C₄-Fluoralkoxy, C₁-C₄-Alkylthio und C₁-C₄-Fluoralkylthio,
umfassend folgende Schritte:
(i) Umsetzung einer Anilinverbindung der Formel II mit einer Zinkorganyl-Verbindung der Formel III, 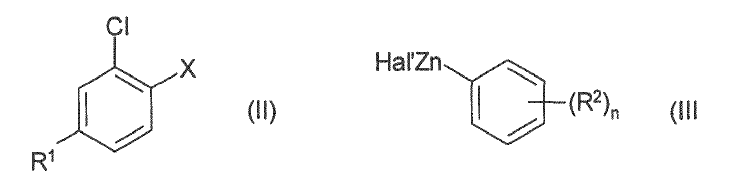 worin
Hal' für Brom oder Chlor steht,
n, R¹ und R² die oben angegebenen Bedeutungen haben, und
X für NH₂ oder einen Rest X¹ oder X² steht, 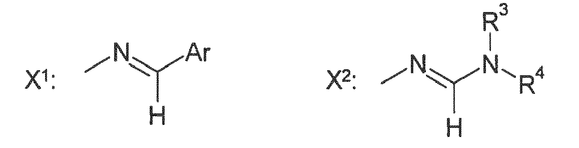
worin
Ar für Phenyl steht, das gegebenenfalls 1, 2, oder 3 Substituenten trägt, die ausgewählt sind unter C₁-C₄-Alkyl und C₁-C₄-Alkoxy, und
R³ und R⁴ unabhängig voneinander für C₁-C₆-Alkyl stehen,
in Gegenwart eines Palladium-Katalysators umfassend Palladium und einen oder mehrere Komplexliganden,
und, wenn X in Formel II für einen Rest X¹ oder X² steht,
(ii) Umsetzung des in Schritt (i) erhaltenen Produkts zu einem 2-Aminobiphenyl der Formel I.

2. Verfahren nach Anspruch 1, wobei X für einen Rest X¹ oder X² steht.

3. Verfahren nach Anspruch 1 oder 2, wobei R¹ für Wasserstoff oder Fluor, R² für Fluor und n für 2 oder 3 stehen.

4. Verfahren nach einem der vorhergehenden Ansprüche, wobei Hal' für Chlor steht.

5. Verfahren nach einem der vorhergehenden Ansprüche, wobei Schritt (i) in einem organischen Lösungsmittel durchgeführt wird, in dem ein Ether enthalten ist.

6. Verfahren nach Anspruch 5, wobei es sich bei dem organischen Lösungsmittel um ein Gemisch aus einem Ether und *N*-Methylpyrrolidion handelt.

7. Verfahren nach einem der vorhergehenden Ansprüche, wobei die Zinkorganyl-Verbindung der Formel III in-situ aus der entsprechenden Grignard-Verbindung erzeugt wird.

8. Verfahren nach einem der vorhergehenden Ansprüche, wobei es sich bei Palladium um Palladium der Oxidationsstufe 0 oder 2 handelt.

9. Verfahren nach einem der vorhergehenden Ansprüche, wobei der Komplexligand entweder monodentates Phosphin der Formel VI und/oder bidentates Phosphin der Formel VII: 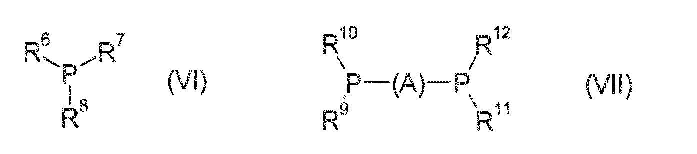 worin R⁶, R⁷, R⁸, R⁹, R¹⁰, R¹¹ und R¹² unabhängig voneinander für C₁-C₈-Alkyl, C₅-C₈-Cycloalkyl, Adamantyl, Aryl-C₁-C₂-alkyl, Ferrocenyl oder Aryl, das gegebenenfalls durch C₁-C₄-Alkyl, C₁-C₄-Alkoxy, Fluor oder Chlor substituiert ist, stehen; und A für Ferrocendiyl oder ein lineares C₂-C₅-Alkandiyl steht, das gegebenenfalls durch C₁-C₈-Alkyl oder C₃-C₆-Cycloalkyl substituiert ist und das gegebenenfalls Teil eines oder zweier mono- oder bicyclischen Ringe ist, welche unsubstituiert oder substituiert sind;
oder wenigstens eine Imidazolylidenverbindung der Formel IX umfaßt: 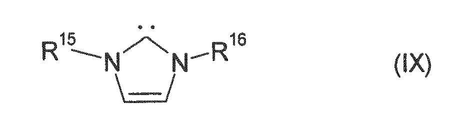 worin R¹⁵ und R¹⁶ unabhängig voneinander für Aryl oder Hetaryl stehen, wobei Aryl und Hetaryl jeweils unsubstituiert sind oder 1, 2, 3 oder 4 Substituenten tragen, die ausgewählt sind unter C₁-C₈-Alkyl und C₃-C₇-Cylcoalkyl.

10. Verbindung der Formel la: 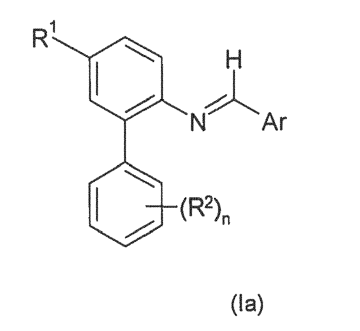 wobei
n für 1, 2 oder 3 steht,
R¹ für Wasserstoff, Cyano oder Fluor steht,
jedes R² jeweils unabhängig voneinander ausgewählt ist unter Cyano, Fluor, C₁-C₄-Alkyl, C₁-C₄-Fluoralkyl, C₁-C₄-Fluoralkoxy, C₁-C₄-Alkylthio und C₁-C₄-Fluoralkylthio, und
Ar für Phenyl steht, das gegebenenfalls 1, 2, oder 3 Substituenten trägt, die ausgewählt sind unter C₁-C₄-Alkyl und C₁-C₄-Alkoxy.

11. Verbindung der Formel Ib: 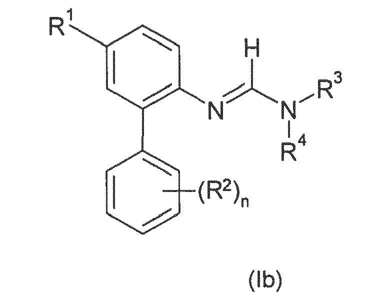 wobei
n für 1, 2 oder 3 steht,
R¹ für Wasserstoff, Cyano oder Fluor steht,
jedes R² jeweils unabhängig voneinander ausgewählt ist unter Cyano, Fluor, C₁-C₄-Fluoralkyl, C₁-C₄-Alkoxy, C₁-C₄-Fluoralkoxy, C₁-C₄-Alkylthio und C₁-C₄-Fluoralkylthio, und
R³ und R⁴ unabhängig voneinander für C₁-C₆-Alkyl stehen.

12. Verbindung der Formel la nach Anspruch 10 oder Ib nach Anspruch 11, wobei R¹ für Wasserstoff oder Fluor, jedes R² jeweils für Fluor, Ar für Phenyl und R³ und R⁴ jeweils für Methyl stehen.

13. Verbindung der Formel la nach Anspruch 10, bei der es sich um 2-Phenylmethylidenamino-(3',4',5'-trifluoro)-biphenyl handelt sowie Verbindung der Formel Ib nach Anspruch 11, bei der es sich um *N,N*-Dimethyl-*N*'-(3',4',5'-trifluorbiphenyl-2-yl)-formamidin handelt.

14. Verfahren zur Herstellung von Pyrazolcarboxamiden der Formel IV 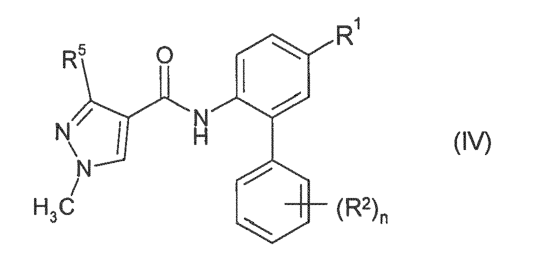 wobei
n für 0, 1, 2 oder 3 steht,
R¹ für Wasserstoff, Cyano oder Fluor steht,
jedes R² jeweils unabhängig voneinander ausgewählt ist unter Cyano, Fluor, C₁-C₄-Alkyl, C₁-C₄-Fluoralkyl, C₁-C₄-Alkoxy, C₁-C₄-Fluoralkoxy, C₁-C₄-Alkylthio und C₁-C₄-Fluoralkylthio, und
R⁵ für Methyl oder Halomethyl steht,
umfassend die Herstellung eines 2-Aminobiphenyls der Formel I nach einem der Ansprüche 1 bis 9 und anschließende *N*-Acylierung des 2-Aminobiphenyls der Formel I mit einer Verbindung der Formel V, 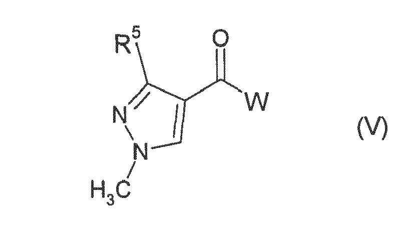 wobei R⁵ die oben genannte Bedeutung hat und W eine Abgangsgruppe bedeutet,
unter Erhalt einer Verbindung der Formel IV.

15. Verfahren nach Anspruch 14, wobei W für Hydroxy oder Halogen steht.

## Claims

1. A process for preparing substituted 2-aminobiphenyls of the formula I 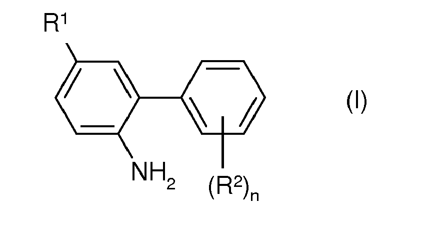 in which
n is 0, 1, 2 or 3,
R¹ is hydrogen, cyano or fluorine, and
each R² is independently selected from cyano, fluorine, C₁-C₄-alkyl, C₁-C₄-fluoroalkyl, C₁-C₄-alkoxy, C₁-C₄-fluoroalkoxy, C₁-C₄-alkylthio and C₁-C₄-fluoroalkylthio,
comprising the following steps:
(i) reacting an aniline compound of the formula II with a zinc organyl compound of the formula III 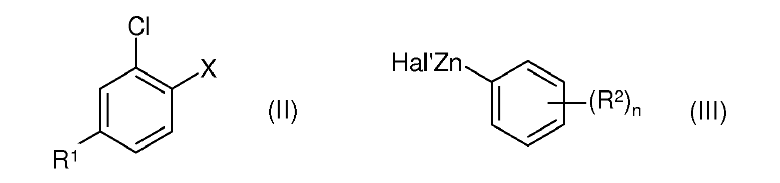 in which
Hal' is bromine or chlorine,
n, R¹ and R² are each as defined above, and
X is NH₂ or an X¹ or X² radical 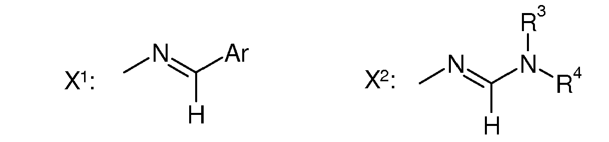
in which
Ar is phenyl which optionally bears 1, 2 or 3 substituents which are selected from C₁-C₄-alkyl and C₁-C₄-alkoxy, and
R³ and R⁴ are each independently C₁-C₆-alkyl,
in the presence of a palladium catalyst comprising palladium and one or more complex ligands,
and, when X in formula II is an X¹ or X² radical,
(ii) converting the product obtained in step (i) to a 2-aminobiphenyl of the formula I.

2. The process according to claim 1, wherein X is an X¹ or X² radical.

3. The process according to claim 1 or 2, wherein R¹ is hydrogen or fluorine, R² is fluorine and n is 2 or 3.

4. The process according to any one of the preceding claims, wherein Hal' is chlorine.

5. The process according to any one of the preceding claims, wherein step (i) is performed in an organic solvent in which an ether is present.

6. The process according to claim 5, wherein the organic solvent is a mixture of an ether and N-methylpyrrolidone.

7. The process according to any one of the preceding claims, wherein the zinc organyl compound of the formula III is generated in situ from the corresponding Grignard compound.

8. The process according to any one of the preceding claims, wherein palladium is palladium in the 0 or 2 oxidation state.

9. The process according to any one of the preceding claims, wherein the complex ligand comprises either monodentate phosphine of the formula VI and/or bidentate phosphine of the formula VII: 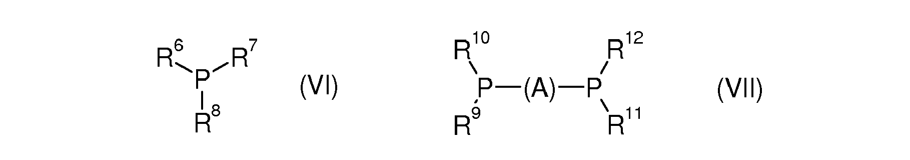 in which R⁶, R⁷, R⁸, R⁹, R¹⁰, R¹¹ and R¹² are each independently C₁-C₈-alkyl, C₅-C₈-cycloalkyl, adamantyl, aryl-C₁-C₂-alkyl, ferrocenyl or aryl which is optionally substituted by C₁-C₄-alkyl, C₁-C₄-alkoxy, fluorine or chlorine; and A is ferrocenediyl or a linear C₂-C₅-alkanediyl which is optionally substituted by C₁-C₈-alkyl or C₃-C₆-cycloalkyl and is optionally part of one or two mono- or bicyclic rings which are unsubstituted or substituted;
or at least one imidazolylidene compound of the formula IX: 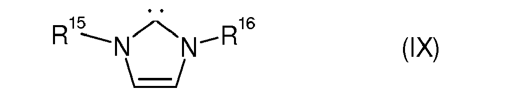 in which R¹⁵ and R¹⁶ are each independently aryl or hetaryl, where aryl and hetaryl are each unsubstituted or bear 1, 2, 3 or 4 substituents selected from C₁-C₈-alkyl and C₃-C₇-cycloalkyl.

10. A compound of the formula Ia: 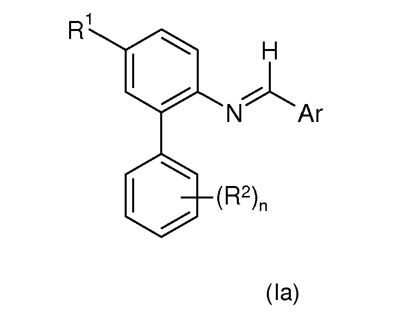 where
n is 1, 2 or 3,
R¹ is hydrogen, cyano or fluorine,
each R² is independently selected from cyano, fluorine, C₁-C₄-alkyl, C₁-C₄-fluoroalkyl, C₁-C₄-fluoroalkoxy, C₁-C₄-alkylthio and C₁-C₄-fluoroalkylthio, and
Ar is phenyl which optionally bears 1, 2, or 3 substituents which are selected from C₁-C₄-alkyl and C₁-C₄-alkoxy.

11. A compound of the formula Ib: 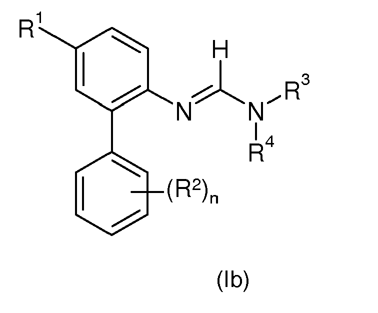 where
n is 1, 2 or 3,
R¹ is hydrogen, cyano or fluorine,
each R² is independently selected from cyano, fluorine, C₁-C₄-fluoroalkyl, C₁-C₄-alkoxy, C₁-C₄-fluoroalkoxy, C₁-C₄-alkylthio and C₁-C₄-fluoroalkylthio, and
R³ and R⁴ are each independently C₁-C₆-alkyl.

12. A compound of the formula Ia according to claim 10 or Ib according to claim 11, wherein R¹ is hydrogen or fluorine, each R² is fluorine, Ar is phenyl and R³ and R⁴ are each methyl.

13. A compound of the formula Ia according to claim 10, which is 2-phenylmethylideneamino(3',4',5'-trifluoro)biphenyl, and a compound of the formula Ib according to claim 11, which is *N,N*-dimethyl-*N*'-(3',4',5'-trifluorobiphenyl-2-yl)formamidine.

14. A process for preparing pyrazolecarboxamides of the formula IV 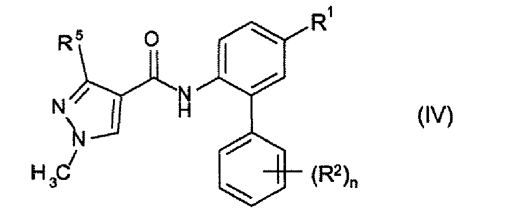 where
n is 0, 1, 2 or 3,
R¹ is hydrogen, cyano or fluorine, and
each R² is independently selected from cyano, fluorine, C₁-C₄-alkyl, C₁-C₄-fluoroalkyl, C₁-C₄-alkoxy, C₁-C₄-fluoroalkoxy, C₁-C₄-alkylthio and C₁-C₄-fluoroalkylthio, and
R⁵ is methyl or halomethyl,
comprising the preparation of a 2-aminobiphenyl of the formula I according to any one of claims 1 to 9 and subsequent *N*-acylation of the 2-aminobiphenyl of the formula I with a compound of the formula V 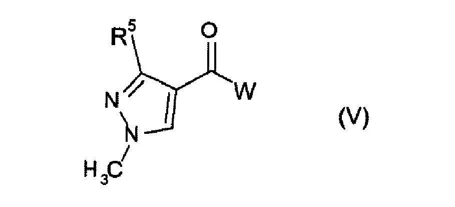 where R⁵ is as defined above and W is a leaving group
to obtain a compound of the formula IV.

15. The process according to claim 14, wherein W is hydroxyl or halogen.

## Revendications

1. Procédé de fabrication de 2-aminobiphényles substitués de formule I 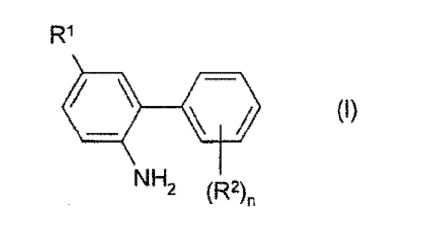 dans laquelle
n représente 0, 1, 2 ou 3,
R¹ représente hydrogène, cyano ou fluor, et
les R² sont chacun choisis indépendamment les uns des autres parmi cyano, fluor, alkyle en C₁-C₄, fluoroalkyle en C₁-C₄, alcoxy en C₁-C₄, fluoroalcoxy en C₁-C₄, alkylthio en C₁-C₄ et fluoroalkylthio en C₁-C₄, comprenant les étapes suivantes :
(i) la mise en réaction d'un composé d'aniline de formule II avec un composé d'organyle de zinc de formule III 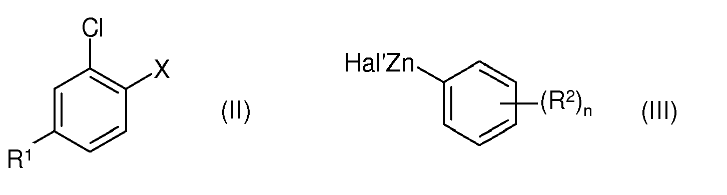 dans lesquelles
Hal' représente brome ou chlore,
n, R¹ et R² ont les significations indiquées précédemment et
X représente NH₂ ou un radical X¹ ou X², 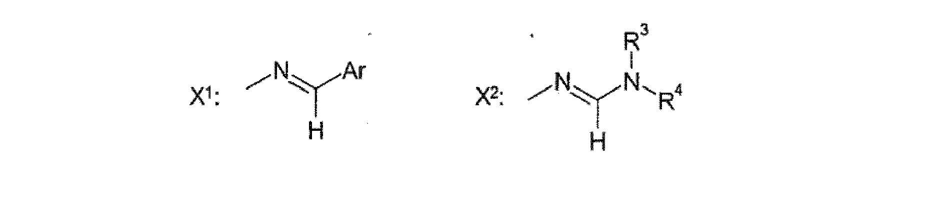
Ar représentant phényle, qui porte éventuellement 1, 2 ou 3 substituants choisis parmi alkyle en C₁-C₄ et alcoxy en C₁-C₄, et
R³ et R⁴ représentant indépendamment l'un de l'autre alkyle en C₁-C₆,
en présence d'un catalyseur de palladium comprenant du palladium et un ou plusieurs ligands complexes,
et, lorsque X dans la formule II représente un radical X¹ ou X²,
(ii) la transformation du produit obtenu à l'étape (i) en un 2-aminobiphényle de formule I.

2. Procédé selon la revendication 1, dans lequel X représente un radical X¹ ou X².

3. Procédé selon la revendication 1 ou 2, dans lequel R¹ représente hydrogène ou fluor, R² représente fluor et n représente 2 ou 3.

4. Procédé selon l'une quelconque des revendications précédentes, dans lequel Hal' représente chlore.

5. Procédé selon l'une quelconque des revendications précédentes, dans lequel l'étape (i) est réalisée dans un solvant organique contenant un éther.

6. Procédé selon la revendication 5, dans lequel le solvant organique est un mélange d'un éther et de N-méthylpyrrolidone.

7. Procédé selon l'une quelconque des revendications précédentes, dans lequel le composé d'organyle de zinc de formule III est formé in situ à partir du composé de Grignard correspondant.

8. Procédé selon l'une quelconque des revendications précédentes, dans lequel le palladium est du palladium de niveau d'oxydation 0 ou 2.

9. Procédé selon l'une quelconque des revendications précédentes, dans lequel le ligand complexe comprend une phosphine monodentate de formule VI et/ou une phosphine bidentate de formule VII : 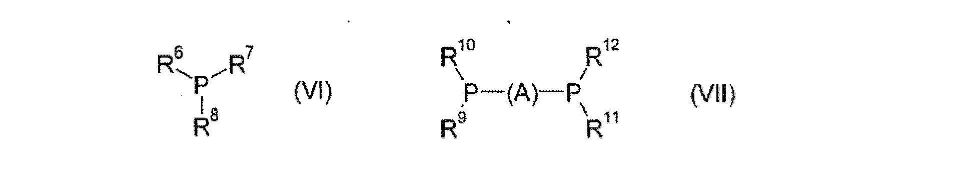 dans lesquelles R⁶, R⁷, R⁸, R⁹, R¹⁰, R¹¹ et R¹² représentent indépendamment les uns des autres alkyle en C₁-C₈, cycloalkyle en C₅-C₈, adamantyle, aryl-alkyle en C₁-C₂, ferrocényle ou aryle, qui est éventuellement substitué par alkyle en C₁-C₄, alcoxy en C₁-C₄, fluor ou chlore ; et A représente ferrocènediyle ou un alcanediyle en C₂-C₅ linéaire, qui est éventuellement substitué par alkyle en C₁-C₈ ou cycloalkyle en C₃-C₆ et qui fait éventuellement partie d'un ou de deux cycles mono- ou bicycliques non substitués ou substitués ;
ou au moins un composé d'imidazolylidène de formule IX : 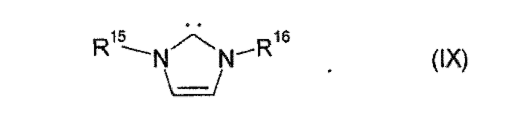 dans laquelle R¹⁵ et R¹⁶ représentent indépendamment l'un de l'autre aryle ou hétaryle ; aryle et hétaryle étant chacun non substitués ou portant 1, 2, 3 ou 4 substituants choisis parmi alkyle en C₁-C₈ et cycloalkyle en C₃-C₇.

10. Composé de formule Ia : 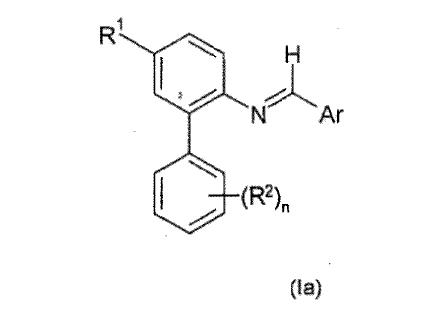 dans laquelle
n représente 1, 2 ou 3,
R¹ représente hydrogène, cyano ou fluor,
les R² sont chacun choisis indépendamment les uns des autres parmi cyano, fluor, alkyle en C₁-C₄, fluoroalkyle en C₁-C₄, fluoroalcoxy en C₁-C₄, alkylthio en C₁-C₄ et fluoroalkylthio en C₁-C₄, et
Ar représente phényle, qui porte éventuellement 1, 2 ou 3 substituants choisis parmi alkyle en C₁-C₄ et alcoxy en C₁-C₄.

11. Composé de formule Ib : 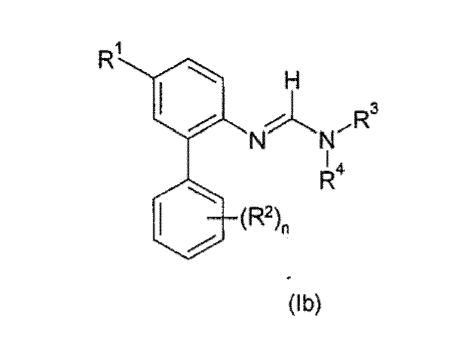 dans laquelle
n représente 1, 2 ou 3,
R¹ représente hydrogène, cyano ou fluor,
les R² sont chacun choisis indépendamment les uns des autres parmi cyano, fluor, fluoroalkyle en C₁-C₄, alcoxy en C₁-C₄, fluoroalcoxy en C₁-C₄, alkylthio en C₁-C₄ et fluoroalkylthio en C₁-C₄, et
R³ et R⁴ représentent indépendamment l'un de l'autre alkyle en C₁-C₆.

12. Composé de formule Ia selon la revendication 10 ou Ib selon la revendication 11, dans lequel R¹ représente hydrogène ou fluor, les R² représentent chacun fluor, Ar représente phényle et R³ et R⁴ représentent chacun méthyle.

13. Composé de formule Ia selon la revendication 10, qui est le 2-phénylméthylidèneamino-(3',4',5'-trifluoro)-biphényle, et composé de formule Ib selon la revendication 11, qui est la *N,N*-diméthyl-*N*'-(3',4',5'-trifluorobiphényl-2-yl)-formamidine.

14. Procédé de fabrication de pyrazolcarboxamides de formule IV 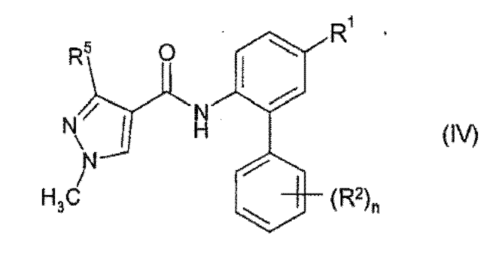 dans laquelle
n représente 0, 1, 2 ou 3,
R¹ représente hydrogène, cyano ou fluor,
les R² sont chacun choisis indépendamment les uns des autres parmi cyano, fluor, alkyle en C₁-C₄, fluoroalkyle en C₁-C₄, alcoxy en C₁-C₄, fluoroalcoxy en C₁-C₄, alkylthio en C₁-C₄ et fluoroalkylthio en C₁-C₄, et
R⁵ représente méthyle ou halométhyle,
comprenant la fabrication d'un 2-aminobiphényle de formule I selon l'une quelconque des revendications 1 à 9, puis la *N*-acylation du 2-aminobiphényle de formule I avec un composé de formule V 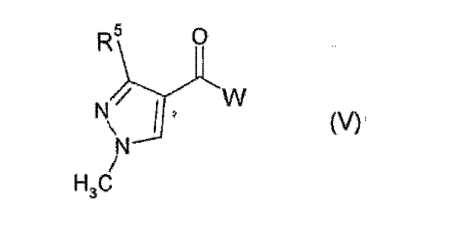 dans laquelle R⁵ a la signification indiquée précédemment et W signifie un groupe partant,
pour obtenir un composé de formule IV.

15. Procédé selon la revendication 14, dans lequel W représente hydroxy ou halogène.
